# EUROPEAN PATENT SPECIFICATION

(11) **EP 2 542 674 B1**
(45) Date of publication and mention of the grant of the patent: **15.01.2014**
(21) Application number: 11711150.0
(22) Date of filing: 02.03.2011
(51) Int. Cl.: C12N 9/24

(54) **GLUCOCEREBROSIDASE MULTIMERS AND USES THEREOF**
GLUCOCEREBROSIDASE-MULTIMERE UND IHRE VERWENDUNG
MULTIMÈRES DE GLUCOCÉRÉBROSIDASE ET LEURS UTILISATIONS

(30) Priority: 02.03.2010 US 309487 P
(43) Date of publication of application: 09.01.2013
(73) Proprietor: Protalix Ltd., 2010000 Carmiel (IL)
(72) Inventor: RUDERFER, Ilya, 21811 Carmiel (IL); KIZHNER, Tali, 2017000 Doar-Na Misgav (IL); SHULMAN, Avidor, 2017500 Doar-Na Misgav (IL); SHAALTIEL, Yoseph, 1790500 Doar-Na HaMovil (IL)
(74) Representative: Gervasi, Gemma
(86) International application number: PCT/IL2011/000210
(87) International publication number: WO 2011/107991

(56) References cited:
- WO-A1-95/01994
- WO-A1-2004/081053
- WO-A2-02/057435
- WO-A2-2009/024977
- KR-A- 20070 065 157

## Description

### FIELD AND BACKGROUND OF THE INVENTION

The present invention, in some embodiments thereof, relates to novel multimeric protein structures and, more particularly, but not exclusively, to multimeric protein structures of glucocerebrosidase and to uses thereof in treating Gaucher disease.

Glucocerebrosidase (D-glucosyl acylsphingosine glucohydrolase, EC 3.2.1.45), also referred to as "GCD", is a lysosomal enzyme that catalyzes the degradation of the fatty substrate, glucosylceramide (GlcCer), in the presence of the activator protein saposin C (SapC). The normal degradation products of GlcCer are glucose and ceramide, which are readily excreted by cells. GCD is a 497-amino acid-long membrane glycoprotein of approximately 65 KDa

Patients with Gaucher disease lack GCD or have dysfunctional GCD, and accordingly, are not able to break down GlcCer. The absence of an active GCD enzyme leads to the accumulation of GlcCer in lysosomes of macrophages. Macrophages affected by the disease become highly enlarged due to the accumulation of GlcCer and are referred to as "Gaucher cells". Gaucher cells accumulate in the spleen, liver, lungs, bone marrow and brain. Symptoms of Gaucher disease may include enlarged liver and spleen, abnormally low levels of red blood cells and platelets, and skeletal complications. Gaucher disease has traditionally been divided into three types based on neurological involvement: Type 1 (non-neuronopathic), Type 2 (acute neuronopathic), and Type 3 (subacute neuronopathic). Gaucher disease is reviewed by Beutler and Grabowski [Gaucher disease, in: Scriver, Beaudet, Sly, and Valle (editors), The Metabolic and Molecular Bases of Inherited Disease, 8th ed., vol. III, New York: McGraw-Hill (2001), pp 3635-3668].

For type 1 patients and most type 3 patients, enzyme replacement treatment with intravenous recombinant glucocerebrosidase can dramatically decrease liver and spleen size, reduce skeletal abnormalities, and reverse other manifestations.

Imiglucerase, which has an amino acid sequence as set forth in SEQ ID NO: 1, is a recombinant DNA-produced analogue of human glucocerebrosidase, which costs approximately $200,000 annually for a single patient and should be continued for life. Velaglucerase alfa, which has an amino acid sequence as set forth in SEQ ID NO: 2, is another recombinant glucocerebrosidase, and was approved by the Food and Drug Administration (FDA) as an alternative treatment in February, 2010.

Taliglucerase alpha, which has an amino acid sequence as set forth in SEQ ID NO: 3, is a plant-derived recombinant glucocerebrosidase. Expression of proteins in plant cell culture is highly efficient, and is not susceptible to contamination by agents such as viruses that are pathological to humans.

WO 2009/024977, by the present assignee, teaches conjugates of a saccharide and a biomolecule, covalently linked therebetween via a non-hydrophobic linker, as well as medical uses utilizing such conjugates.

Basu and Glew [J Biol Chem 1986, 260:13067-13073] describes activation of glucocerebrosidase by ganglioside molecules, which is associated by formation of a complex consisting of 50 % glucocerebrosidase and 50 % ganglioside, the complex comprising two glucocerebrosidase molecules.

Additional background art includes Stenson et al. [Hum Mutat 2003, 21:577-581], Beutler et al. [Mol Med 1994, 1:82-92], Theophilus et al. [Am J Hum Genet 1989, 45:212-225], Chabás et al. [J Med Genet 1995; 32:740-742], Abrahamov et al. [Lancet 1995, 346:1000-1003], Montfort et al. [Hum Mutat 2004, 23:567-575], Bendele et al. [Toxicological Sciences 1998, 42:152-157], U.S. Patent Nos. 5,256804, 5,580757 and 5,766,897, International Patent Application PCT/NL2007/050684 (published as WO 2008/075957), and Seely & Richey [J Chromatography A 2001, 908:235-241].

### SUMMARY OF THE INVENTION

The present inventors have observed that glucocerebrosidase (GCD) activity at neutral pH (e.g., in plasma) and under acidic conditions (such as exist in lysosomes) is compromised with time and accordingly have recognized a need for GCD that exhibits an improved and lasting activity. To this effect, the present inventors have designed and successfully prepared and practiced novel multimeric forms of native GCD and have surprisingly uncovered that multimeric forms of native glucocerebrosidase exhibit a longer lasting activity under both lysosomal conditions and in a serum environment, which allows for an enhanced activity of the protein *in vivo.*

According to an aspect of some embodiments of the resent invention there is provided a multimeric protein structure comprising at least two glucocerebrosidase molecules being covalently linked to one another via a linking moiety, the multimeric protein structure featuring a characteristic selected from the group consisting of:
(a) a glucocerebrosidase activity upon subjecting the multimeric protein structure to human plasma conditions for one hour, which is at least 10 % higher than an activity of native glucocerebrosidase upon subjecting the native glucocerebrosidase to the human plasma conditions for one hour;
(b) a glucocerebrosidase activity which decreases upon subjecting the multimeric protein structure to human plasma conditions for one hour by a percentage which is at least 10 % less than the percentage by which an activity of the native glucocerebrosidase decreases upon subjecting the native glucocerebrosidase to the human plasma conditions for one hour;
(c) a glucocerebrosidase activity which remains substantially unchanged upon subjecting the multimeric protein structure to human plasma conditions for one hour;
(d) a glucocerebrosidase activity, upon subjecting the multimeric protein structure to lysosomal conditions for 4 days, which is at least 10 % higher than an activity of native glucocerebrosidase upon subjecting the native glucocerebrosidase to the lysosomal conditions for 4 days;
(e) a glucocerebrosidase activity which decreases upon subjecting the multimeric protein structure to lysosomal conditions for one day by a percentage which is at least 10 % less than the percentage by which an activity of the native glucocerebrosidase decreases upon subjecting the native glucocerebrosidase to the lysosomal conditions for one day;
(f) a glucocerebrosidase activity which remains substantially unchanged upon subjecting the multimeric protein structure to lysosomal conditions for one day; and
(g) a circulating half-life in a physiological system which is higher by at least 20 % than a circulating half-life of the native glucocerebrosidase.

According to some embodiments of the invention, the multimeric protein structure is characterized by a glucocerebrosidase activity upon subjecting the multimeric protein structure to human plasma conditions for one hour, which is at least 10-fold an activity of native glucocerebrosidase upon subjecting the native glucocerebrosidase to the human plasma conditions for one hour.

According to some embodiments of the invention, the linking moiety is not present in native glucocerebrosidase.

According to an aspect of some embodiments of the invention there is provided a multimeric protein structure comprising at least two glucocerebrosidase molecules being covalently linked to one another via a linking moiety, wherein the linking moiety is not present in native glucocerebrosidase.

According to some embodiments of the invention, the multimeric protein structure is featuring a characteristic selected from the group consisting of:
(a) a glucocerebrosidase activity upon subjecting the multimeric protein structure to human plasma conditions for one hour, which is at least 10 % higher than an activity of native glucocerebrosidase upon subjecting the native glucocerebrosidase to the human plasma conditions for one hour;
(b) a glucocerebrosidase activity which decreases upon subjecting the multimeric protein structure to human plasma conditions for one hour by a percentage which is at least 10 % less than the percentage by which an activity of the native glucocerebrosidase decreases upon subjecting the native glucocerebrosidase to the human plasma conditions for one hour;
(c) a glucocerebrosidase activity which remains substantially unchanged upon subjecting the multimeric protein structure to human plasma conditions for one hour;
(d) a glucocerebrosidase activity, upon subjecting the multimeric protein structure to lysosomal conditions for 4 days, which is at least 10 % higher than an activity of native glucocerebrosidase upon subjecting the native glucocerebrosidase to the lysosomal conditions for 4 days;
(e) a glucocerebrosidase activity which decreases upon subjecting the multimeric protein structure to lysosomal conditions for one day by a percentage which is at least 10 % less than the percentage by which an activity of the native glucocerebrosidase decreases upon subjecting the native glucocerebrosidase to the lysosomal conditions for one day;
(f) a glucocerebrosidase activity which remains substantially unchanged upon subjecting the multimeric protein structure to lysosomal conditions for one day; and
(g) a circulating half-life in a physiological system which is higher by at least 20 % than a circulating half-life of the native glucocerebrosidase.

According to some embodiments of the invention, the multimeric protein structure is characterized by a glucocerebrosidase activity upon subjecting the multimeric protein structure to human plasma conditions for one hour, which is at least 10-fold an activity of native glucocerebrosidase upon subjecting the native glucocerebrosidase to the human plasma conditions for one hour.

According to some embodiments of the invention, the circulating half-life of the multimeric protein structure which is higher than a circulating half-life of the native glucocerebrosidase, is higher by at least 50 % than the circulating half-life of the native glucocerebrosidase.

According to some embodiments of the invention, the multimeric protein structure as described herein is characterized by a glucocerebrosidase activity in an organ upon administration of the multimeric protein structure to a vertebrate, the organ being selected from the group consisting of a liver, a spleen, a kidney, a lung, a bone marrow and blood.

According to some embodiments of the invention, the multimeric protein structure as described herein comprises two glucocerebrosidase molecules, the protein structure being a dimeric protein structure.

According to some embodiments of the invention, the glucocerebrosidase is a human glucocerebrosidase.

According to some embodiments of the invention, the glucocerebrosidase is a plant recombinant glucocerebrosidase.

According to some embodiments of the invention, the glucocerebrosidase has an amino acids sequence selected from the group consisting of SEQ ID NO: 1, SEQ ID NO: 2, and SEQ ID NO: 3.

According to some embodiments of the invention, the linking moiety comprises a poly(alkylene glycol).

According to some embodiments of the invention, the poly(alkylene glycol) comprises at least two functional groups, each functional group forming a covalent bond with one of the glucocerebrosidase molecules.

According to some embodiments of the invention, the at least two functional groups are terminal groups of the poly(alkylene glycol).

According to some embodiments of the invention, the at least one linking moiety has a general formula:

-X₁-(CR₁R₂-CR₃R₄-Y)n-X₂-

wherein each of X₁ and X₂ is a functional group that forms a covalent bond with at least one glucocerebrosidase molecule;
Y is O, S or NR₅;
n is an integer from 1 to 200; and
each of R₁, R₂, R₃, R₄ and R₅ is independently selected from the group consisting of hydrogen, alkyl, cycloalkyl, alkenyl, alkynyl, alkoxy, hydroxy, oxo, thiol and thioalkoxy.

According to some embodiments of the invention, at least one of the functional groups forms an amide bond with a glucocerebrosidase molecule.

According to some embodiments of the invention, n is an integer from 1 to 15.

According to some embodiments of the invention, n is an integer from 4 to 10.

According to an aspect of some embodiments of the invention there is provided a pharmaceutical composition comprising a multimeric protein structure as described herein and a pharmaceutically acceptable carrier.

According to some embodiments of the invention, the pharmaceutical further comprises an ingredient selected from the group consisting of glucose, a saccharide comprising a glucose moiety, nojirimycin, and derivatives thereof.

According to an aspect of some embodiments of the invention there is provided a multimeric protein structure as described herein, for use as a medicament.

According to some embodiments of the invention, the medicament is for treating Gaucher disease.

According to an aspect of some embodiments of the invention there is provided a multimeric protein structure as described herein, for use in treating Gaucher disease.

According to an aspect of some embodiments of the invention there is provided a multimeric protein structure as described herein, method of treating Gaucher disease, the method comprising administering to a subject in need thereof a therapeutically effective amount of the multimeric protein structure as described herein, thereby treating the Gaucher disease.

According to an aspect of some embodiments of the invention there is provided a process of preparing the multimeric protein structure as described herein, the process comprising reacting glucocerebrosidase with a cross-linking agent which comprises the linking moiety and at least two reactive groups.

According to some embodiments of the invention, conditions for the reacting are selected such that the multimeric protein structure formed by cross-linking the glucocerebrosidase is a dimer.

According to some embodiments of the invention, the reactive groups comprise a leaving group.

According to some embodiments of the invention, the reactive group reacts with an amine group to form an amide bond.

According to some embodiments of the invention, each of the reactive groups is capable of forming a covalent bond between the linking moiety and at least one glucocerebrosidase molecule.

According to some embodiments of the invention, a molar ratio of the cross-linking agent to the glucocerebrosidase is in a range of from 5:1 to 500:1.

According to some embodiments of the invention, the molar ratio is in a range of from 75:1 to 300:1.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

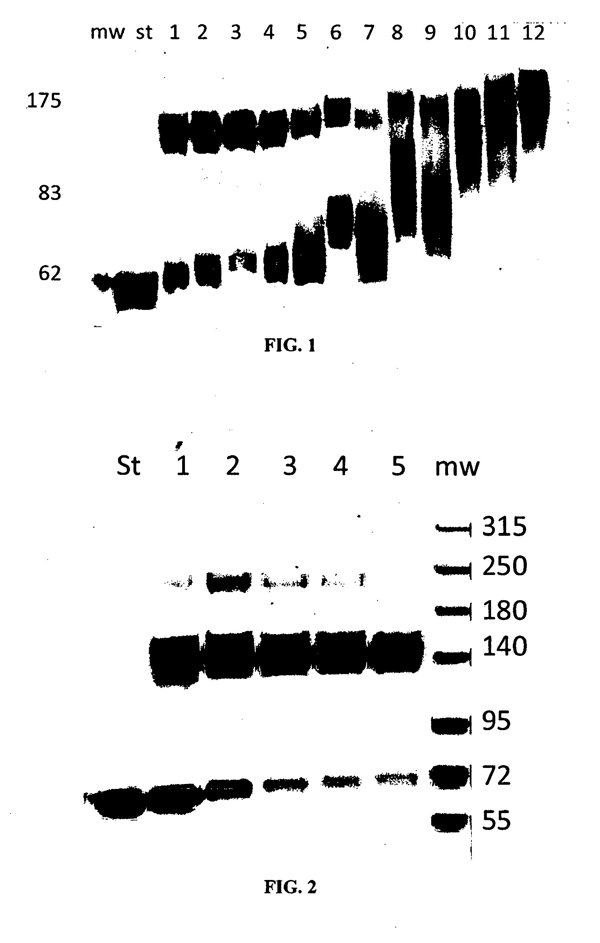

FIG. 1 presents a scan of an SDS-PAGE gel showing plant recombinant glucocerebrosidase which was reacted with 50 (lanes 1, 3, 5, 7, 9 and 11) or 100 (lanes 2, 4, 6, 8, 10 and 12) molar equivalents of bis-NHS-PEG₅ (lanes 1-2), bis-NHS-PEG₈ (lanes 3-4), bis-NHS-PEG₂₁ (lanes 5-6), bis-NHS-PEG₄₅ (lanes 7-8), bis-NHS-PEG₆₈ (lanes 9-10), and bis-NHS-PEG₁₃₆ (lanes 11-12) bis-*N*-hydroxysuccinimide-poly(ethylene glycol) (bis-NHS-PEG) reagent, as well as molecular weight markers (mw) (molecular weights of markers are shown on left) and non-reacted plant recombinant glucecerebrosidase standard (st);
FIG. 2 presents a scan of an SDS-PAGE gel showing plant recombinant glucocerebrosidase which was reacted with 25 (lane 1), 50 (lane 2), 75 (lane 3), 100 (lane 4) and 200 (lane 5) molar equivalents of bis-NHS-PEG₅, as well as molecular weight markers (mw) (molecular weights of markers are shown on right) and non-reacted plant recombinant glucecerebrosidase standard (St);

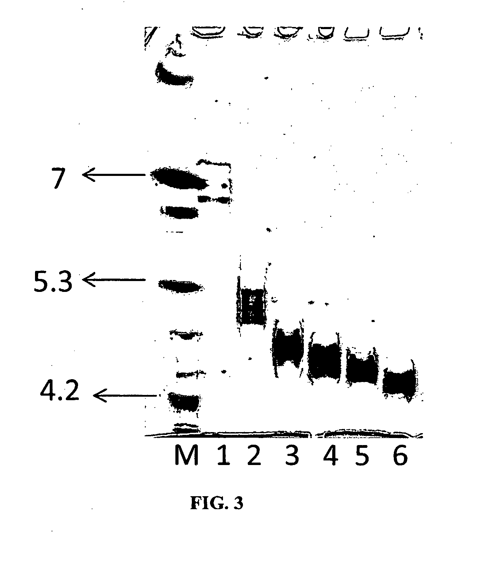

FIG. 3 presents a scan of an isoelectric focusing gel showing plant recombinant glucocerebrosidase which was reacted with 25 (lane 2), 50 (lane 3), 75 (lane 4), 100 (lane 5) and 200 (lane 6) molar equivalents of bis-NHS-PEG₅, as well as pH markers (M) and non-reacted plant recombinant glucocerebrosidase (lane 1) (arrows show pH values for various bands);

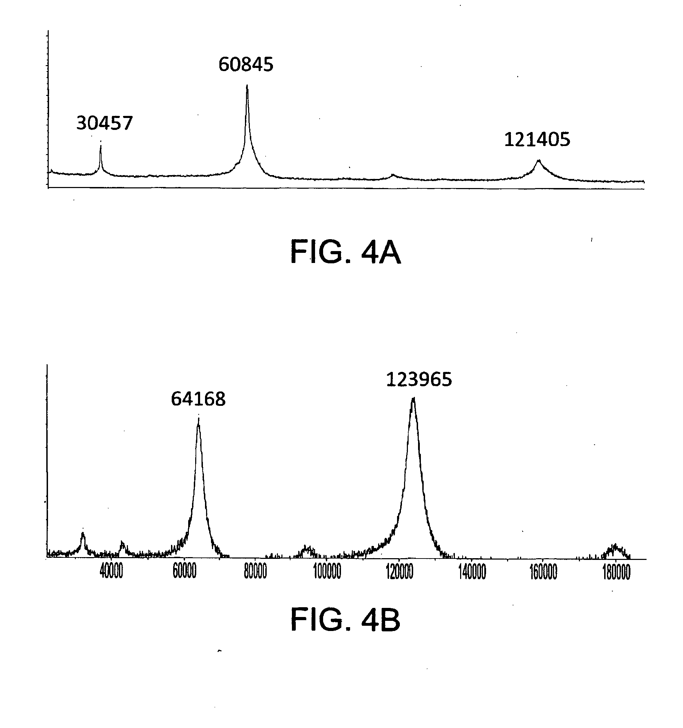

FIGs. 4A and 4B present a MALDI-TOF mass spectroscopy spectrum of plant recombinant glucocerebrosidase (FIG. 4A) and of plant recombinant glucocerebrosidase cross-linked by 75 molar equivalents of bis-NHS-PEG₅ (FIG. 4B; x-axis indicates m/z values, and m/z values of peaks are shown);

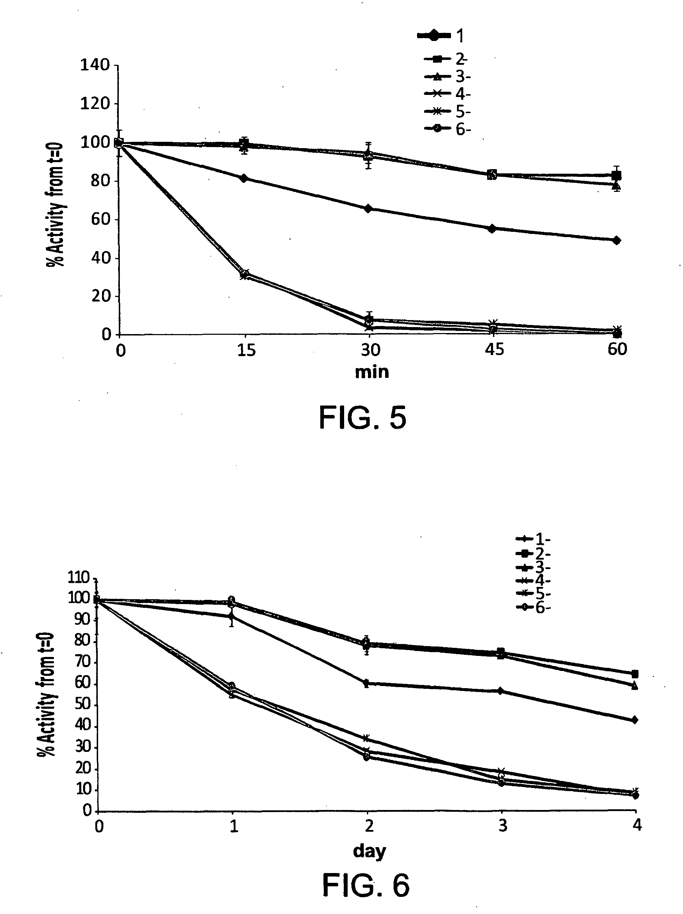

FIG. 5 is a graph showing the activity of plant recombinant glucocerebrosidase from two different batches (4 and 6), plant recombinant glucocerebrosidase PEGylated with 50 molar equivalents of methoxy-capped PEG₈-NHS (5), and plant recombinant glucocerebrosidase cross-linked with 25 molar equivalents (1), 75 molar equivalents (2), or 200 molar equivalents (3) of bis-NHS-PEG₅, as a function of incubation time in human plasma at 37 °C (activity is normalized to value at time = 0);
FIG. 6 is a graph showing the activity of plant recombinant glucocerebrosidase from two different batches (4 and 6), plant recombinant glucocerebrosidase PEGylated with 50 molar equivalents of methoxy-capped PEG₈-NHS (5), plant recombinant glucocerebrosidase cross-linked with 25 molar equivalents (1), 75 molar equivalents (2), or 200 molar equivalents (3) of bis-NHS-PEG₅, as a function of incubation time under simulated lysosomal conditions (citrate phosphate buffer, pH 4.6, 37 °C) (activity is normalized to value at time = 0); and

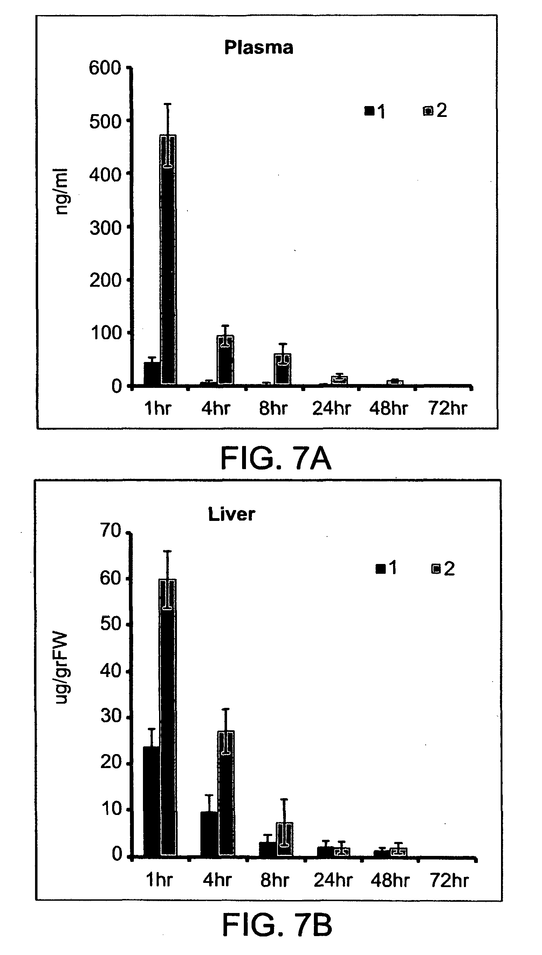

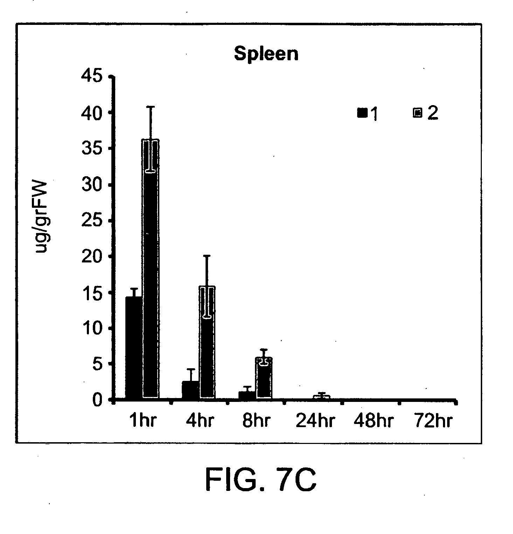

FIGs. 7A-7C are bar graphs showing the activity of plant recombinant glucocerebrosidase (1) and plant recombinant glucocerebrosidase cross-linked with 75 molar equivalents of bis-NHS-PEG₅ (2) in the plasma (FIG. 7A), liver (FIG. 7B) and spleen (FIG. 7C) of male mice as a function of time following injection.

### DESCRIPTION OF SPECIFIC EMBODIMENTS OF THE INVENTION

The present invention, in some embodiments thereof, relates to novel multimeric protein structures and, more particularly, but not exclusively, to multimeric protein structures of glucocerebrosidase and to uses thereof in treating Gaucher disease.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

Deficiencies of a lysosomal protein (e.g., defects in a lysosomal protein or absence of a lysosomal protein) can cause considerable harm to the health of a subject (a lysosomal storage disease). Enzyme replacement therapy (ERT), in which the deficient protein is administered to a patient, has been used in attempts to treat lysosomal storage diseases. However, administration of the deficient protein does not necessarily result in a considerable and/or persistent increase in the activity of the protein *in vivo.*

Gaucher disease is an example of an autosomal recessive (inherited) lysosomal storage disease which can cause a wide range of systemic symptoms. A deficiency of the lysosomal enzyme glucocerebrosidase due to mutation causes a glycolipid known as glucocerebroside to accumulate in the body (e.g., in the spleen, liver, kidneys, brain and bone marrow), particularly in white blood cells. This accumulation leads to an impairment of their proper function. Two enzyme replacement therapies (ERTs) are available to functionally compensate for glucocerebrosidase deficiency. Imiglucerase (Cerezyme^{®}, Genzyme) and velaglucerase alfa (VPRIV^{®}, Shire) are both recombinant forms of the human glucocerebrosidase enzyme. These enzymes are difficult to manufacture and as such are very expensive.

As shown herein, glucocerebrosidase activity at neutral pH (e.g., in plasma) is rapidly compromised. Thus, for example, glucocerebrosidase used in ERT would have little ability to hydrolyze glucocerebroside in target organs and/or cells of Gaucher patients, as the glucocerebrosidase would be compromised in the blood before reaching its target.

Moreover, as further shown herein, even under acidic conditions (such as exist in lysosomes), the activity of glucocerebrosidase is gradually compromised, although at a slower rate than at higher pH levels.

Motivated by a need to solve the compromised activity of glucocerebrosidase, the present inventors have searched for modified forms of glucocerebrosidase (GCD), which exhibit longer lasting activity in general, and longer lasting activity in serum in particular. The present inventors have surprisingly uncovered that multimeric forms of native glucocerebrosidase exhibit a longer lasting activity under both lysosomal conditions and in a serum environment, which allows for an enhanced activity of the protein *in vivo.*

The present inventors have demonstrated a formation of multimeric forms of glucocerebrosidase which exhibit an improved performance by means of cross-linking native glucocerebrosidase molecules, via formation of new covalent linkages between glucocerebrosidase molecules. Formation of linkages between molecules of glucocerebrosidase has heretofore never been described.

Referring now to the drawings, Figures 1-4 show that an exemplary GCD, plant recombinant human glucocerebrosidase (prh-GCD), reacted with exemplary cross-linking agents comprising N-hydroxysuccinimide moieties to form covalently linked multimers, primarily dimers. Figure 1 shows that the cross-linking is more efficient when relatively short cross-linking reagents are used.

Figure 5 shows that the cross-linked prh-GCD exhibits a longer lasting activity than either non-PEGylated native GCD or non-cross-linked PEGylated GCD in human plasma. Figure 6 shows that the cross-linked prh-GCD exhibits a longer lasting activity than either non-PEGylated native GCD or non-cross-linked PEGylated GCD under simulated lysosomal conditions. Figures 5 and 6 both show that the increase in stability is due to cross-linking rather than PEGylation, and that it is dependent on the conditions used for cross-linking. Figures 7A-7C show that following injection, the cross-linked prh-GCD exhibits higher activity in the plasma, spleen and liver of mice than does an equal amount of non-cross-linked prh-GCD.

The results presented herein show that multimeric protein structures formed by covalently cross-linking glucocerebrosidase molecules are characterized by a more stable enzymatic activity under physiologically relevant conditions, as compared to the native glucocerebrosidase.

Thus, as exemplified herein, the covalently-linked multimeric protein structure may exhibit an activity which is higher than an activity of native glucocerebrosidase, as a result of the activity of the native glucocerebrosidase decaying more rapidly over time than the activity of the cross-linked multimeric protein structure.

Hence, according to an aspect of some embodiments of the present invention there is provided a multimeric protein structure comprising at least two glucocerebrosidase molecules being covalently linked to one another via a linking moiety. According to some embodiments, the multimeric protein structure features a more stable activity than that of native glucocerebrosidase, as described in detail below.

Herein, the phrase "glucocerebrosidase molecule" refers to a glucocerebrosidase protein having a monomeric form, for example, containing a single polypeptide. The polypeptide may include non-peptidic substituents (e.g., one or more saccharide moieties). Thus, in a multimeric protein structure comprising at least two glucocerebrosidase molecules being covalently linked to one another, each glucocerebrosidase molecule is a monomer of the multimeric protein structure.

Herein, the term "native" with respect to glucocerebrosidase encompasses proteins comprising an amino acid sequence substantially identical (i.e., at least 95 % homology, optionally at least 99 % homology, and optionally 100 %) to an amino acid sequence of a naturally occurring glucocerebrosidase protein as defined herein.

As used herein, "glucocerebrosidase" refers to any protein which exhibits an enzymatic activity catalyzing the hydrolysis the β-glucosidic linkage of glucocerebroside.

According to optional embodiments, "glucocerebrosidase" refers to E.C. 3.2.1.45. In some embodiments, "glucocerebrosidase" refers exclusively to a lysosomal protein (a protein naturally occurring in lysosomes).

The glucocerebrosidase of embodiments of the invention can be purified (e.g., from plants or animal tissue) or generated by recombinant DNA technology.

The glucocerebrosidase of embodiments of the invention can be of any human, animal or plant source, provided no excessively adverse immunological reaction is induced upon *in vivo* administration (e.g., plant to human).

Optionally, the glucocerebrosidase is a human glucocerebrosidase (e.g., a recombinant human glucocerebrosidase), for example, in order to facilitate optimal biocompatibility for administration to human subjects. Recombinant human glucocerebrosidase is commercially available, for example, as imiglucerase and velaglucerase alfa.

Herein, "human glucocerebrosidase" refers to a glucocerebrosidase comprising an amino acid sequence substantially identical (e.g., as described hereinabove) to an amino acid sequence of a glucocerebrosidase protein (as defined herein) which naturally occurs in humans.

In some embodiments, the glucocerebrosidase is a plant recombinant glucocerebrosidase. Exemplary glucocerebrosidase include plant recombinant human glucocerebrosidase. Plant recombinant human glucocerebrosidase produced from transgenic carrot cells is known in the art as taliglucerase alpha.

Examples of glucocerebrosidase include, without limitation, glucocerebrosidase having an amino acid sequence as set forth in any of SEQ ID NO: 1, SEQ ID NO: 2 and SEQ ID NO: 3.

In some embodiments, the glucocerebrosidase has an amino acid sequence as set forth in SEQ ID NO:3.

It is to be noted that heretofore, there are no reports of native glucocerebrosidase that has a multimeric form. However, the term "native" with respect to glucocerebrosidase encompasses any from of native glucocerebrosidase, including any monomeric and multimeric form.

It is to be further noted that in the case of a native glucocerebrosidase which has a multimeric form, the multimeric structure described herein typically includes covalent bonds between the GCD monomers which are not present in a multimeric native GCD.

A native glucocerebrosidase may be a protein isolated from a natural source, or a recombinantly produced protein (e.g., derived from mammalian cells, plant cells, yeast cells, fungal cells, bacterial cells, insect cells and the like).

Herein, the phrase "naturally occurring" with respect to glucocerebrosidase or any other protein refers to a protein in a form which occurs in nature (e.g., in an organism), with respect to the protein's amino acid sequence.

Post-translational modifications (e.g., glycosylation) of naturally occurring glucocerebrosidase proteins (e.g., in an organism which expresses the naturally occurring glucocerebrosidase protein) may be present, absent or modified in the native form of glucocerebrosidase referred to herein. A native form of glucocerebrosidase (e.g., a recombinantly produced glucocerebrosidase) may optionally comprise different post-translational modifications than those of the naturally occurring glucocerebrosidase, provided that the native form of the glucocerebrosidase retains a substantially similar amino acid sequence and structure and\or function as the naturally occurring glucocerebrosidase, as described herein.

Optionally, the multimeric protein structure described herein is a dimeric structure, comprising two glucocerebrosidase molecules covalently linked to one another.

Alternatively, the multimeric protein structure comprises more than two glucocerebrosidase molecules. For example, the multimeric protein structure may be a trimer, a tetramer, a pentamer, a hexamer, a heptamer or an octamer comprised of glucocerebrosidase molecules.

The multimeric protein structures described herein comprise covalent bonds which link the glucocerebrosidase molecules therein, and which are absent from native glucocerebrosidase.

Optionally, the linking moiety which links the glucocerebrosidase molecules is a moiety which is not present in native glucocerebrosidase (e.g., a synthetic linking moiety).

Thus, for example, the linking moiety is optionally a moiety which is covalently attached to a side chain, an N-terminus or a C-terminus, or a moiety related to post-translational modifications (e.g., a saccharide moiety), of a glucocerebrosidase molecule, as well as to a side chain, an N-terminus or a C-terminus, or a moiety related to post-translational modifications (e.g., a saccharide moiety) of another glucocerebrosidase molecule. Exemplary such linking moieties are described in detail hereinunder.

Alternatively, the linking moiety forms a part of the glucocerebrosidase molecules being linked (e.g., a part of a side chain, N-terminus or C-terminus or moiety related to post-translational modifications (e.g., saccharide moiety) of a glucocerebrosidase molecule, as well as of a side chain, an N-terminus or a C-terminus or a moiety related to post-translational modifications (e.g., saccharide moiety) of another glucocerebrosidase molecule).

Thus, for example, the linking moiety can be a covalent bond (e.g., an amide bond) between a functional group of a side chain, N-terminus , C-terminus or moiety related to post-translational modifications of a glucocerebrosidase molecule (e.g., an amine), and a complementary functional group of a side chain, N-terminus , C-terminus or moiety related to post-translational modifications of another glucocerebrosidase molecule (e.g., carboxyl), such a covalent bond being absent from native glucocerebrosidase, although the functional groups being linked are themselves present in a glucocerebrosidase molecule. Other covalent bonds, such as, for example, an ester bond (between a hydroxy group and a carboxyl); a thioester bond; an ether bond (between two hydroxy groups); a disulfide bond (between two thiol groups); a thioether bond; an anhydride bond (between two carboxyls); a thioamide bond; a carbamate or thiocarbamate bond, are also contemplated.

Optionally, the linking moiety is devoid of a disulfide bond. However, a linking moiety which includes a disulfide bond at a position such that the disulfide bond is not essential for forming a link between glucocerebrosidase molecules (e.g., cleavage of the disulfide bond does not cleave the link between the molecules) is within the scope of this embodiment of the invention. A potential advantage of linking moiety devoid of a disulfide bond is that it is not susceptible to cleavage by mild reducing conditions, as are disulfide bonds.

Optionally, the linking moiety is a non-peptidic moiety (e.g., the linking moiety does not consist of an amide bond, an amino acid, a dipeptide, a tripeptide, an oligopeptide or a polypeptide). A potential advantage of linking moiety which is a non-peptidic moiety is that it is not susceptible to cleavage by proteases and peptidases (e.g., such as are present *in vivo*)*.*

Alternatively, the linking moiety may be, or may comprise, a peptidic moiety (e.g., an amino acid, a dipeptide, a tripeptide, an oligopeptide or a polypeptide).

Optionally, the linking moiety is not merely a linear extension of any of the glucocerebrosidase molecules attached thereto (i.e., the N-terminus and C-terminus of the peptidic moiety is not attached directly to the C-terminus or N-terminus of any of the glucocerebrosidase molecules).

Alternatively, the linking moiety is formed by direct covalent attachment of an N-terminus of a glucocerebrosidase molecule with a C-terminus of another glucocerebrosidase molecule, so as to produce a fused polypeptide which is a non-native form of glucocerebrosidase.

However, in some embodiments, the covalent linking of glucocerebrosidase molecules described herein is in a form other than direct linkage of an N-terminus to a C-terminus.

The linking moiety is also referred to herein as a cross-linking moiety. The linking of glucocerebrosidase molecules by a linking moiety is referred to herein as "cross-linking".

The cross-linking moiety can be a covalent bond, a chemical atom or group (e.g., a C(=O)-O- group, -O-, -S-, NR-, -N=N-, -NH-C(=O)-NH-, -NH-C(=O)-, -NH-C(=O)-O- and the like) or a bridging moiety (composed of a chain of chemical groups).

A bridging moiety can be, for example, a polymeric or oligomeric group.

A "bridging moiety" refers to a multifunctional moiety (e.g., biradical, triradical, etc.) that is attached to side chains, moieties related to post-translational modifications (e.g., saccharide moieties) and/or termini (i.e., N-termini, C-termini) of two or more of the glucocerebrosidase molecules.

According to some embodiments, the linking moiety is not a covalent bond, a chemical atom or group, but is rather a bridging moiety.

As exemplified herein in the Examples section, relatively short linking moieties (e.g., PEG₅, PEG₈) are particularly effective at cross-linking between different glucocerebrosidase molecules, in comparison to longer linking moieties (e.g., PEG₂₁, PEG₄₅, PEG₆₈, PEG₁₃₆).

Hence, according to some embodiments, the linking moiety is no more than 60 atoms long, optionally no more than 40 atoms long, optionally no more than 30 atoms long, and optionally no more than 20 atoms long.

Herein, the length of a linking moiety (when expressed as a number of atoms) refers to length of the backbone of the linking moiety, i.e., the number atoms forming a linear chain between residues of each of two glucocerebrosidase molecules linked via the linking moiety.

Optionally, the linking moiety is below a certain size, so as to avoid an unnecessarily excessive part of the linking moiety in the formed cross-linked protein structure, which may interfere with the function of the protein, and/or so as to avoid complications and/or inefficiency in a synthesis of the linking moiety. In addition, a large linking moiety may also be less effective at cross-linking between different glucocerebrosidase molecules, as described herein with respect to PEG₂₁, PEG₄₅, PEG₆₈, and PEG₁₃₆ linkers, in comparison to smaller linking moieties.

Hence, according to some embodiments, each linking moiety is characterized by a molecular weight of less than 5 KDa, optionally less than 3 KDa, optionally less than 2 KDa, optionally less than 1 KDa, and optionally less than 0.5 KDa.

In order to facilitate cross-linking, the linking moiety is optionally substantially flexible, wherein the bonds in the backbone of the linking moiety are mostly rotationally free, for example, single bonds which are not coupled to a double bond (e.g., unlike an amide bond) and wherein rotation is not sterically hindered. Optionally, at least 70 %, optionally at least 80 %, and optionally at least 90 % (e.g., 100 %) of the bonds in the backbone of the linking moiety are rotationally free.

In some embodiments, the linking moiety comprises a poly(alkylene glycol) chain.

The phrase "poly(alkylene glycol)", as used herein, encompasses a family of polyether polymers which share the following general formula: -O-[(CH₂)ₘ-O-]ₙ-, wherein m represents the number of methylene groups present in each alkylene glycol unit, and n represents the number of repeating units, and therefore represents the size or length of the polymer. For example, when m = 2, the polymer is referred to as a polyethylene glycol, and when m = 3, the polymer is referred to as a polypropylene glycol.

In some embodiments, m is an integer greater than 1 (e.g., m = 2, 3, 4, etc.).

Optionally, m varies among the units of the poly(alkylene glycol) chain. For example, a poly(alkylene glycol) chain may comprise both ethylene glycol (m=2) and propylene glycol (m=3) units linked together.

The poly(alkylene glycol) optionally comprises at least two functional groups (e.g., as described herein), each functional group forming a covalent bond with one of the glucocerebrosidase molecules. The functional groups are optionally terminal groups of the poly(alkylene glycol), such that the entire length of the poly(alkylene glycol) lies between the two functional groups and represents the length of the linking moiety.

The phrase "poly(alkylene glycol)" also encompasses analogs thereof, in which the oxygen atom is replaced by another heteroatom such as, for example, S, -NH- and the like. This term further encompasses derivatives of the above, in which one or more of the methylene groups composing the polymer are substituted. Exemplary substituents on the methylene groups include, but are not limited to, alkyl, cycloalkyl, alkenyl, alkynyl, alkoxy, hydroxy, oxo, thiol and thioalkoxy, and the like.

The phrase "alkylene glycol unit", as used herein, encompasses a -(CH₂)ₘ-O-group or an analog thereof, as described hereinabove, which forms the backbone chain of the poly(alkylene glycol), wherein the (CH₂)ₘ (or analog thereof) is bound to a heteroatom belonging to another alkylene glycol unit or to an glucocerebrosidase moiety (in cases of a terminal unit), and the O (or heteroatom analog thereof) is bound to the (CH₂)ₘ (or analog thereof) of another alkylene glycol unit, or to a functional group which forms a bond with a glucocerebrosidase molecule.

An alkylene glycol unit may be branched, such that it is linked to 3 or more neighboring alkylene glycol units, wherein each of the 3 or more neighboring alkylene glycol units are part of a poly(alkylene glycol) chain. Such a branched alkylene glycol unit is linked via the heteroatom thereof to one neighboring alkylene glycol unit, and heteroatoms of the remaining neighboring alkylene glycol units are each linked to a carbon atom of the branched alkylene glycol unit. In addition, a heteroatom (e.g., nitrogen) may bind more than one carbon atom of an alkylene glycol unit of which it is part, thereby forming a branched alkylene glycol unit (e.g., [(-CH₂)ₘ]₂N- and the like).

In exemplary embodiments, at least 50 % of alkylene glycol units are identical, e.g., they comprise the same heteroatoms and the same m values as one another. Optionally, at least 70 %, optionally at least 90 %, and optionally 100 % of the alkylene glycol units are identical. In exemplary embodiments, the heteroatoms bound to the identical alkylene glycol units are oxygen atoms. In further exemplary embodiments, m is 2 for the identical units.

In one embodiment, the linker is a single, straight chain linker, preferably being polyethylene glycol (PEG).

As used herein, the term "poly(ethylene glycol)" describes a poly(alkylene glycol), as defined hereinabove, wherein at least 50 %, at least 70 %, at least 90 %, and preferably 100 %, of the alkylene glycol units are -CH₂CH₂-O-. Similarly, the phrase "ethylene glycol units" is defined herein as units of -CH2CH₂O-.

According to optional embodiments, the linking moiety comprises a poly(ethylene glycol) or analog thereof, having a general formula:

-X₁-(CR₁R₂-CR₃R₄-Y)ₙ-X₂-

wherein each of X₁ and X₂ is a functional group (e.g., as described herein) that forms a covalent bond with at least one glucocerebrosidase molecule;
Y is O, S or NR₅ (optionally O);
n is an integer, optionally from 1 to 200, although higher values of n are also contemplated; and
each of R₁, R₂, R₃, R₄, and R₅ is independently selected from the group consisting of hydrogen, alkyl, cycloalkyl, alkenyl, alkynyl, alkoxy, hydroxy, oxo, thiol and thioalkoxy.

In some embodiments, n is no more than 100, optionally no more than 50, and optionally no more than 25. In some embodiments, n is no more than 15, and optionally no more than 10.

In some embodiments, n is at least 2, and optionally at least 3, and optionally at least 4. In some embodiments, n is from 4 to 10. Thus, in some embodiments, n can be 4, 5, 6, 7, 8, 9 or 10, whereby higher values, such as 11, 12, 13, 14, 14, 16, 17, 18, 19, 20, 25, 30, 35, 40, 45, 50 and any integers therebetween, are also contemplated.

The poly(ethylene glycol) or analog thereof may optionally comprise a copolymer, for example, wherein the CR₁R₂-CR₃R₄-Y units in the above formula are not all identical to one another.

In some embodiments, at least 50 % of CR₁R₂-CR₃R₄-Y units are identical. Optionally, at least 70 %, optionally at least 90 %, and optionally 100 % of the CR₁R₂-CR₃R₄-Y units are identical.

Optionally, the linking moiety is branched, for example, such that for one or more CR₁R₂-CR₃R₄-Y units in the above formula, at least of one of R₁, R₂, R₃, R₄, and R₅ is -(CR₁R₂-CR₃R₄-Y)ₚX₃-, wherein R₁-R₄ and Y are as defined hereinabove, p is an integer as defined herein for n (e.g., from 1 to 200), and X₃ is as defined herein for X₁ and X₂.

The functional groups may optionally form a bond such as, but not limited to, an amide bond, an amine bond, an ester bond, and/or an ether bond.

For example, the functional group may optionally comprise a carbonyl group which forms an amide bond with a nitrogen atom in a glucocerebrosidase molecule (e.g., in a lysine residue or N-terminus), or an ester bond with an oxygen atom in a glucocerebrosidase molecule (e.g., in a serine, threonine or tyrosine residue).

Alternatively or additionally, the functional group may optionally comprise a heteroatom (e.g., N, S, O) which forms an amide bond, ester bond or thioester bond with a carbonyl group in a glucocerebrosidase molecule (e.g., in a glutamate or aspartate residue or in a C-terminus).

Alternative or additionally, the functional group may comprise an alkyl or aryl group attached to a glucocerebrosidase molecule (e.g., to a heteroatom in the glucocerebrosidase).

Alternatively or additionally, the functional group may optionally comprise a nitrogen atom which forms an amine bond with an alkyl group in a glucocerebrosidase molecule, or the glucocerebrosidase may optionally comprise a nitrogen atom which forms an amine bond with an alkyl group in the functional group. Such an amine bond may be formed by reductive amination (e.g., as described hereinbelow).In some embodiments, at least one of the functional groups forms an amide bond with a glucocerebrosidase molecule (e.g., with a lysine residue therein).

The functional groups may be identical to one another or different.

In some embodiments, at least one of the functional groups is attached to one functionality of a polypeptide (e.g., an amine group of a lysine residue or N-terminus), and at least one of the functional groups is attached to a different functionality of a polypeptide (e.g., a thiol group of a cysteine residue).

As exemplified in the Examples herein, the multimeric protein structure described herein exhibits a highly stable activity in human plasma conditions and/or in lysosomal conditions.

As used herein, "stable activity" means that the activity of the protein is long-lasting when the protein is exposed to conditions such as are described herein.

As used herein, the phrase "human plasma conditions" refers to human plasma as a medium, at a temperature of 37 °C.

As used herein, the phrase "lysosomal conditions" refers to an aqueous solution having a pH of 4.6 as a medium (e.g., a citrate phosphate buffer described herein), at a temperature of 37 °C.

Enhanced stability under lysosomal conditions is advantageous because the lysosome is a target for replacement therapy for glucocerebrosidase, as lysosomes are the normal location for glucocerebrosidase activity in a body, and lysosomal conditions (e.g., acidic pH) represent optimal conditions for activity of glucocerebrosidase.

Without being bound by any particular theory, it is believed that enhanced stability in serum-like conditions (e.g., the human plasma conditions described herein) is also advantageous because enhanced stability of glucocerebrosidase allows more of the glucocerebrosidase to reach a target organ and/or cells.

According to optional embodiments, the stable activity of the multimeric protein structure in human plasma conditions is such that the multimeric protein structure exhibits, upon being subjected to human plasma conditions for one hour, a glucocerebrosidase activity which is at least 10 % higher, optionally at least 20 % higher, optionally at least 50 % higher, and optionally at least 100 % higher, than a glucocerebrosidase activity of native glucocerebrosidase upon subjecting the native glucocerebrosidase to the human plasma conditions for one hour. In some embodiments, the activity of the multimeric protein structure is at least twice (100 % higher), optionally at least 3-fold (200 % higher), optionally at least 5-fold (400 % higher), optionally at least 10-fold (900 % higher), optionally at least 20-fold, optionally at least 50-fold, and optionally at least 100-fold the activity of the native glucocerebrosidase, upon being subjected to human plasma conditions for one hour.

Alternatively or additionally, the multimeric protein structure exhibits a glucocerebrosidase activity which decreases upon subjecting the protein structure to human plasma conditions for one hour by a percentage which is at least 10 % less, optionally at least 20 % less, optionally at least 50 % less, optionally at least 80 % less, optionally at least 90 % less, optionally at least 95 % less, and optionally at least 99 % less, than the percentage by which a corresponding activity of the native glucocerebrosidase decreases upon subjecting the native glucocerebrosidase to human plasma conditions for one hour.

It is to be appreciated that by exhibiting an activity which decreases at a lower rate than that of native glucocerebrosidase, the multimeric protein structure will, over time, eventually exhibit considerably more activity than the native glucocerebrosidase, even if the multimeric protein structure is initially moderately less active than the native protein.

It is to be understood that herein, a decrease which is "10 % less" than a decrease of 50 % refers to a decrease of 45 % (45 being 10 % less than 50), and not to a decrease of 40 % (50% - 10%).

Alternatively or additionally, the stable activity of the multimeric protein structure in human plasma conditions is such that a glucocerebrosidase activity of the multimeric protein structure remains substantially unchanged upon subjecting the multimeric protein structure to human plasma conditions for one hour, and optionally for 2, 4 or even 6 hours.

As used herein, the phrase "substantially unchanged" refers to a level (e.g., of activity) which remains in a range of from 50 % to 150 % of the initial level, and optionally a level which remains at least 60 %, optionally at least 70 %, optionally at least 80 %, and optionally at least 90 % of the initial level.

Optionally, the stable activity of the multimeric protein structure in lysosomal conditions is such that the multimeric protein structure exhibits, upon being subjected to lysosomal conditions for a predetermined time period (e.g., one day, two days, 3 days, 4 days, one week), a glucocerebrosidase activity which is at least 10 % higher, optionally 20 % higher, optionally 50 % higher, and optionally 100 % higher, than an activity of native glucocerebrosidase upon subjecting the native glucocerebrosidase to the lysosomal conditions for the same predetermined time period.

Alternatively or additionally, the multimeric protein structure exhibits a glucocerebrosidase activity which decreases upon subjecting the protein structure to lysosomal conditions for a predetermined time period (e.g., one day, 2 days, 3 days, 4 days, one week), by a percentage which is at least 10 % less, optionally 20 % less, optionally 50 % less, and optionally 80 % less, than the percentage by which a corresponding activity of the native glucocerebrosidase decreases upon subjecting the native glucocerebrosidase to lysosomal conditions for the same time period.

Alternatively or additionally, the stable activity of the multimeric protein structure in lysosomal conditions is such that a glucocerebrosidase activity of the multimeric protein structure remains substantially unchanged upon subjecting the multimeric protein structure to lysosomal conditions for one day, for 2 days, for 3 days, for 4 days, and/or for one week.

The glucocerebrosidase activity described herein is a biological activity which is characteristic of glucocerebrosidase (e.g., a catalytic activity characteristic of glucocerebrosidase, such as hydrolysis of a terminal β-glucosyl moiety of a substrate).

In some embodiments, a catalytic activity of glucocerebrosidase is characterized by a rate of catalysis at saturation (i.e., a *Vₘₐₓ* value).

Alternatively, the glucocerebrosidase activity is a therapeutic activity (e.g., an enzymatic activity having a therapeutic effect), such as a therapeutic activity in the context of Gaucher disease. Optionally, the therapeutic activity is determined in experimental animals (e.g., Gaucher mice), and optionally in human Gaucher patients.

Techniques for determining an activity of glucocerebrosidase will be known to a skilled person. Typically, the glucocerebrosidase (i.e., native glucocerebrosidase or a multimeric protein structure described herein) is contacted with a compound recognized in the art as a substrate of glucocerebrosidase, and the degree of activity is then determined quantitatively. Compounds which allow for particularly convenient detection of glucocerebrosidase activity are known in the art and are commercially available.

In some embodiments, glucocerebrosidase activity is determined by assaying hydrolysis of 4-methylumbelliferyl-β-D-glucopyranoside (e.g., as described in the Examples section herein).

In some embodiments, glucocerebrosidase activity is determined by assaying hydrolysis of *p*-nitrophenyl-β-D-glucopyranoside (e.g., as described in the Examples section herein).

In some embodiments, glucocerebrosidase activity is determined by assaying hydrolysis of glucocerebroside or a fluorescent derivative thereof (e.g., glucocerebroside-nitrobenzoxadiazole).

When comparing an activity of a multimeric protein structure described herein with an activity of native glucocerebrosidase, the native glucocerebrosidase preferably comprises glucocerebrosidase substantially identical (e.g., with respect to amino acid sequence and glycosylation pattern) to the glucocerebrosidase molecules comprised by the multimeric structure.

According to some embodiments, the multimeric protein structure is characterized by a circulating half-life in a physiological system (e.g., blood, serum and/or plasma of a human or laboratory animal) which is higher (e.g., at least 20 %, at least 50 % higher, at least 100 % higher, at least 400 % higher, at least 900 % higher) than a circulating half-life of native glucocerebrosidase.

An increased circulating half-life may optionally be associated with a higher *in vitro* stability (e.g, as described herein), a higher *in vivo* stability (e.g, resistance to metabolism) and/or with other factors (e.g., reduced renal clearance).

Circulating half-lives can be determined by taking samples (e.g., blood samples, tissue samples) from physiological systems (e.g., humans, laboratory animals) at various intervals, and determining a level of glucocerebrosidase in the sample, using techniques known in the art.

Optionally, the half-life is calculated as a terminal half-life, wherein half-life is the time required for a concentration (e.g., a blood concentration) to decrease by 50 % after pseudo-equilibrium of distribution has been reached. The terminal half-life may be calculated from a terminal linear portion of a time vs. log concentration, by linear regression of time vs. log concentration (see, for example, Toutain & Bousquet-Melou [J Vet Pharmacol Ther 2004, 27:427-39]). Thus, the terminal half-life is a measure of the decrease in drug plasma concentration due to drug elimination and not of decreases due to other reasons, and is not necessarily the time necessary for the amount of the administered drug to fall by one half.

Determining a level of glucocerebrosidase (e.g., in the form of the multimeric protein structure or as native glucocerebrosidase) may comprise detecting the physical presence of glucocerebrosidase molecules (e.g., via an antibody against glucocerebrosidase) and/or detecting a level of a glucocerebrosidase activity (e.g., as described herein).

According to some embodiments, the multimeric protein structure is characterized by a glucocerebrosidase activity in an organ (e.g., spleen, heart, kidney, brain, liver, lungs, and bone marrow) upon administration (e.g., intravenous administration) of the protein structure to a vertebrate (e.g., a human, a mouse), for example, a vertebrate with a glucocerebrosidase deficiency (e.g., a human Gaucher disease patient, a Gaucher mouse). Optionally, the glucocerebrosidase activity in the organ is higher than a glucocerebrosidase activity of native glucocerebrosidase in the organ, upon an equivalent administration to a vertebrate.

The activity in an organ may be a function of uptake of the glucocerebrosidase and/or retention of glucocerebrosidase activity following uptake.

Optionally, glucocerebrosidase activity in the organ is determined 1 hour after administration, optionally 2 hours after administration, optionally 4 hours after administration, optionally 6 hour after administration, and optionally 8 hours after administration optionally 12 hour after administration, and optionally 16 hours after administration and optionally 24 hours after administration.

In some embodiments, the multimeric protein structure is characterized by an enhanced glucocerebrosidase activity in an organ such as, but not limited to, liver, spleen, kidneys, lungs, bone marrow and blood. In exemplary embodiments, the organ is liver, spleen and blood. A level of activity in blood is optionally determined according to a level of activity in serum. In some embodiments, the multimeric protein structure is characterized by an enhanced glucocerebrosidase activity in an organ after administration (as described herein) which is at least 20 % higher, optionally at least 50 % higher, optionally at least 100 % higher, and optionally at least 300 % higher, than the activity of native glucocerebrosidase after an equivalent administration.

As noted hereinabove, the present inventors have devised and successfully prepared and practiced stabilized forms of glucocerebrosidase by means of multimeric structures of cross-linked glucocerebrosidase molecules.

As exemplified in the Examples section herein, a multimeric protein structure described herein may be conveniently prepared by reacting glucocerebrosidase with a cross-linking agent.

Hence, according to another aspect of embodiments of the invention, there is provided a process of preparing a multimeric protein structure described herein. The process comprises reacting glucocerebrosidase (i.e., a plurality of glucocerebrosidase molecules), so as to introduce at least one linking moiety which covalently links at least two glucocerebrosidase molecules.

Optionally, the linking moiety is a bond (e.g., an amide bond, a disulfide bond) which links one glucocerebrosidase molecule to another glucocerebrosidase molecule. Optionally, the bond is introduced by using suitable conditions and/or reagents. For example, reagents which are suitable for forming an amide bond from a carboxylic acid group and an amine group are known in the art.

Optionally, the linking moiety is a moiety which is not derived from a part of the glucocerebrosidase. For example, the linking moiety may be an oligomer, a polymer, a residue of a small molecule (e.g., an amino acid).

In some embodiments, the linking moiety is introduced by reacting the glucocerebrosidase molecules with a cross-linking agent which comprises the linking moiety (e.g., as described herein) and at least two reactive groups.

In some embodiments, the cross-linking agent is reacted with the glucocerebrosidase at a molar ratio in a range of from 5:1 to 500:1 (cross-linking agent: glucocerebrosidase). In exemplary embodiments, the molar ratio is in a range of from 25:1 to 200:1.

According to some embodiments, the molar ratio is at least 50:1, optionally in a range of from 50:1 to 400:1, and optionally in a range of from 75:1 to 300:1 (e.g., about 100:1, about 200:1).

According to some embodiments, the molar ratio is 25:1, 30:1, 40:1, 50:1, 60:1, 70:1, 75:1, 80:1, 90:1, 100:1, 110:1, 120:1, 130:1, 140:1, 150:1, 160:1, 170:1, 180:1, 190:1, 200:, 250:1, or 300:1, with any values between the above-indicated values being also contemplated.

Optionally, the conditions for the reaction (e.g., concentration of reactants, introduction and/or concentration of an organic co-solvent, polarity and/or pH of solvent) are selected such that a multimeric protein structure obtained by the reaction is a dimer (e.g., wherein at least 50 % of the obtained multimeric protein structures are a dimer). Alternatively or additionally, conditions may be selected such that a multimeric structure other than a dimer is obtained (e.g., a trimer, a tetramer, a hexamer).

For example, under conditions wherein glucocerebrosidase is in a monomeric form, a degree of cross-linking between molecules may depend strongly on a concentration of the glucocerebrosidase (e.g., the reaction may be a second-order reaction), with formation of a multimer (e.g., a dimer) being favored in the presence of high concentrations of glucocerebrosidase.

In comparison, under conditions wherein glucocerebrosidase aggregates to form a multimeric form, the multimeric protein structure obtained may be relatively independent of the glucocerebrosidase concentration (e.g., the reaction may be a zero-order reaction). Thus, for example, when cross-linking under conditions in which glucocerebrosidase is a dimer, the obtained multimeric protein structure may be a dimer. Alternatively or additionally, other structures (e.g., a tetramer, a hexamer) may be obtained.

The process optionally further comprises purifying the cross-linked protein, for example, removing excess cross-linking agent. Common purification methods may be used, such as dialysis and/or ultra-filtration using appropriate cut-off membranes and/or additional chromatographic steps, including size exclusion chromatography, ion exchange chromatography, affinity chromatography, hydrophobic interaction chromatography, and the like.

The reactive group is selected suitable for undergoing a chemical reaction that leads to a bond formation with a complementary functionality in the glucocerebrosidase. Optionally, each reactive group is capable of forming a covalent bond between the linking moiety described herein and at least one glucocerebrosidase molecule (e.g., so as to form a functional group bound to the polypeptide, as described herein).

The reactive groups of a cross-linking agent may be identical to one another or different.

As used herein, the phrase "reactive group" describes a chemical group that is capable of undergoing a chemical reaction that typically leads to a bond formation. The bond, according to the present embodiments, is preferably a covalent bond (e.g., for each of the reactive groups). Chemical reactions that lead to a bond formation include, for example, nucleophilic and electrophilic substitutions, nucleophilic and electrophilic addition reactions, alkylations, addition-elimination reactions, cycloaddition reactions, rearrangement reactions and any other known organic reactions that involve a functional group, as well as combinations thereof.

The reactive group may optionally comprise a non-reactive portion (e.g., an alkyl) which may serve, for example, to attach a reactive portion of the reactive group to a linking moiety (e.g., poly(alkylene glycol) or analog thereof) described herein.

The reactive group is preferably selected so as to enable its conjugation to glucocerebrosidase. Exemplary reactive groups include, but are not limited to, carboxylate (e.g., -CO₂H), thiol (-SH), amine (-NH₂), halo, azide (-N₃), isocyanate (-NCO), isothiocyanate (-N=C=S), hydroxy (-OH), carbonyl (e.g., aldehyde), maleimide, sulfate, phosphate, sulfonyl (e.g. mesyl, tosyl), etc. as well as activated groups, such as *N*-hydroxysuccinimide (NHS) (e.g. NHS esters), sulfo-*N*-hydroxysuccinimide, anhydride, acyl halide (-C(=O)-halogen) etc.

In some embodiments, the reactive group comprises a leaving group, such as a leaving group susceptible to nucleophilic substitution (e.g., halo, sulfate, phosphate, carboxylate, N-hydroxysuccinimide).

Optionally, the reactive group may be in an activated form thereof.

As used herein, the phrase "activated form" describes a derivative of a chemical group (e.g., a reactive group) which is more reactive than the chemical group, and which is thus readily capable of undergoing a chemical reaction that leads to a bond formation. The activated form may comprise a particularly suitable leaving group, thereby facilitating substitution reactions. For example, a -C(=O)-NHS group (N-hydroxysuccinimide ester, or -C(=O)-O-succinimide) is a well-known activated form of -C(=O)OH, as NHS (N-hydroxysuccinimide) can be reacted with a -C(=O)OH to form -C(=O)-NHS, which readily reacts to form products characteristic of reactions involving -C(=O)OH groups, such as amides and esters.

The reactive group can be attached to the rest of the linking moiety (e.g., a poly(alkylene glycol) or analog thereof) via different groups, atoms or bonds. These may include an ether bond [e.g., -O-alkyl-], an ester bond [e.g., -O-C(=O)-alkyl-], a carbamate [e.g., O-C(=O)-NH-alkyl-], an amide bond, etc. Thus, a variety of terminal groups can be employed.

The following are non-limiting examples of the different groups that may constitute a reactive group as described herein: -CH₂CO₂H, -CH₂CH₂CO₂H, -CH₂CH₂SH, -CH₂CH₂NH₂, -CH₂CH₂N₃, -CH₂CH₂NCO, -CH₂-C(=O)-NHS, -CH₂CH₂-C(=O)-NHS, -C(=O)-CH₂-C(=O)-NHS, -CH₂CH₂-NHC(=O)CH₂CH₂-maleimide, etc.

The number of methylene groups in each of the above reactive groups is merely exemplary, and may be varied.

The reactive group may also comprise the heteroatom at the end of a poly(alkylene glycol) chain (e.g., -OH).

In exemplary embodiments of the present invention, the reactive group comprises a carboxylate (e.g., an activated carboxylate such as an N-hydroxysuccinimide ester).

Optionally, the reactive group reacts with an amine group in the glucocerebrosidase (e.g., in a lysine residue and/or an N-terminus) to form an amide bond.

In some embodiments, the reaction of the reactive group comprises reductive amination, wherein an amine group reacts with an aldehyde group to form an imine, and the imine is reduced (e.g., by addition of a reducing agent, such as sodium cyanoborohydride) to form an amine bond. The reactive group may be an amine group which reacts with an aldehyde group of the glucocerebrosidase (e.g., on a saccharide moiety), or the reactive group may be an aldehyde group which reacts with an amine group of the glucocerebrosidase (e.g., on a lysine residue). Optionally, a saccharide moiety of the glucocerebrosidase (i.e., a glycan) is oxidized by an oxidizing agent to form an aldehyde group, prior to reaction of the reactive group with the glucecerebrosidase. For example, reaction of a saccharide with sodium periodate may be used to produce a pair of aldehyde groups in a saccharide moiety.

In some embodiments, at least one of the reactive groups is selected so as to react with one functionality of a glucocerebrosidase molecule (e.g., an amine group of a lysine residue or N-terminus), and at least one of the reactive groups is selected so as to react with a different functionality of a glucocerebrosidase molecule (e.g., a thiol group of a cysteine residue).

As used herein, the terms "amine" and "amino" refer to either a -NR'R" group, wherein R' and R" are selected from the group consisting of hydrogen, alkyl, cycloalkyl, heteroalicyclic (bonded through a ring carbon), aryl and heteroaryl (bonded through a ring carbon). R' and R" are bound via a carbon atom thereof. Optionally, R' and R" are selected from the group consisting of hydrogen and alkyl comprising 1 to 4 carbon atoms. Optionally, R' and R" are hydrogen.

As used herein throughout, the term "alkyl" refers to a saturated aliphatic hydrocarbon including straight chain and branched chain groups. Preferably, the alkyl group has 1 to 20 carbon atoms. Whenever a numerical range; *e.g*., "1-20", is stated herein, it implies that the group, in this case the alkyl group, may contain 1 carbon atom, 2 carbon atoms, 3 carbon atoms, etc., up to and including 20 carbon atoms. More preferably, the alkyl is a medium size alkyl having 1 to 10 carbon atoms. Most preferably, unless otherwise indicated, the alkyl is a lower alkyl having 1 to 4 carbon atoms. The alkyl group may be substituted or unsubstituted. When substituted, the substituent group can be, for example, cycloalkyl, alkenyl, alkynyl, aryl, heteroaryl, heteroalicyclic, halo, hydroxy, alkoxy, aryloxy, thiohydroxy, thioalkoxy, thioaryloxy, sulfinyl, sulfonyl, cyano, nitro, azide, phosphonyl, phosphinyl, oxo, carbonyl, thiocarbonyl, urea, thiourea, O-carbamyl, N-carbamyl, O-thiocarbamyl, N-thiocarbamyl, C-amido, N-amido, C-carboxy, O-carboxy, sulfonamido, and amino, as these terms are defined herein.

A "cycloalkyl" group refers to an all-carbon monocyclic or fused ring (i.e., rings which share an adjacent pair of carbon atoms) group wherein one of more of the rings does not have a completely conjugated pi-electron system. Examples, without limitation, of cycloalkyl groups are cyclopropane, cyclobutane, cyclopentane, cyclopentene, cyclohexane, cyclohexadiene, cycloheptane, cycloheptatriene, and adamantane. A cycloalkyl group may be substituted or unsubstituted. When substituted, the substituent group can be, for example, alkyl, alkenyl, alkynyl, aryl, heteroaryl, heteroalicyclic, halo, hydroxy, alkoxy, aryloxy, thiohydroxy, thioalkoxy, thioaryloxy, sulfinyl, sulfonyl, cyano, nitro, azide, phosphonyl, phosphinyl, oxo, carbonyl, thiocarbonyl, urea, thiourea, O-carbamyl, N-carbamyl, O-thiocarbamyl, N-thiocarbamyl, C-amido, N-amido, C-carboxy, O-carboxy, sulfonamido, and amino, as these terms are defined herein.

An "alkenyl" group corresponds to an alkyl group which consists of at least two carbon atoms and at least one carbon-carbon double bond.

An "alkynyl" group corresponds to an alkyl group which consists of at least two carbon atoms and at least one carbon-carbon triple bond.

An "aryl" group refers to an all-carbon monocyclic or fused-ring polycyclic (*i.e*., rings which share adjacent pairs of carbon atoms) groups having a completely conjugated pi-electron system. Examples, without limitation, of aryl groups are phenyl, naphthalenyl and anthracenyl. The aryl group may be substituted or unsubstituted. When substituted, the substituent group can be, for example, alkyl, alkenyl, alkynyl, cycloalkyl, aryl, heteroaryl, heteroalicyclic, halo, hydroxy, alkoxy, aryloxy, thiohydroxy, thioalkoxy, thioaryloxy, sulfinyl, sulfonyl, cyano, nitro, azide, phosphonyl, phosphinyl, oxo, carbonyl, thiocarbonyl, urea, thiourea, O-carbamyl, N-carbamyl, O-thiocarbamyl, N-thiocarbamyl, C-amido, N-amido, C-carboxy, O-carboxy, sulfonamido, and amino, as these terms are defined herein.

A "heteroaryl" group refers to a monocyclic or fused ring (i.e., rings which share an adjacent pair of atoms) group having in the ring(s) one or more atoms, such as, for example, nitrogen, oxygen and sulfur and, in addition, having a completely conjugated pi-electron system. Examples, without limitation, of heteroaryl groups include pyrrole, furane, thiophene, imidazole, oxazole, thiazole, pyrazole, pyridine, pyrimidine, quinoline, isoquinoline and purine. The heteroaryl group may be substituted or unsubstituted. When substituted, the substituent group can be, for example, alkyl, alkenyl, alkynyl, cycloalkyl, aryl, heteroaryl, heteroalicyclic, halo, hydroxy, alkoxy, aryloxy, thiohydroxy, thioalkoxy, thioaryloxy, sulfinyl, sulfonyl, cyano, nitro, azide, phosphonyl, phosphinyl, oxo, carbonyl, thiocarbonyl, urea, thiourea, O-carbamyl, N-carbamyl, O-thiocarbamyl, N-thiocarbamyl, C-amido, N-amido, C-carboxy, O-carboxy, sulfonamido, and amino, as these terms are defined herein.

A "heteroalicyclic" group refers to a monocyclic or fused ring group having in the ring(s) one or more atoms such as nitrogen, oxygen and sulfur. The rings may also have one or more double bonds. However, the rings do not have a completely conjugated pi-electron system. The heteroalicyclic may be substituted or unsubstituted. When substituted, the substituted group can be, for example, lone pair electrons, alkyl, alkenyl, alkynyl, cycloalkyl, aryl, heteroaryl, heteroalicyclic, halo, hydroxy, alkoxy, aryloxy, thiohydroxy, thioalkoxy, thioaryloxy, sulfinyl, sulfonyl, cyano, nitro, azide, phosphonyl, phosphinyl, oxo, carbonyl, thiocarbonyl, urea, thiourea, O-carbamyl, N-carbamyl, O-thiocarbamyl, N-thiocarbamyl, C-amido, N-amido, C-carboxy, O-carboxy, sulfonamido, and amino, as these terms are defined herein. Representative examples are piperidine, piperazine, tetrahydrofuran, tetrahydropyran, morpholine and the like.

A "hydroxy" group refers to an -OH group.

An "azide" group refers to a -N=N⁺=N⁻ group.

An "alkoxy" group refers to both an -O-alkyl and an -O-cycloalkyl group, as defined herein.

An "aryloxy" group refers to both an -O-aryl and an -O-heteroaryl group, as defined herein.

An "ether" refers to both an alkoxy and an aryloxy group, wherein the group is linked to an alkyl, alkenyl, alkynyl, cycloalkyl, aryl, heteroaryl or heteroalicyclic group.

An ether bond describes a -O- bond.

A "thiohydroxy" or "thiol" group refers to a -SH group.

A "thioalkoxy" group refers to both an -S-alkyl group, and an -S-cycloalkyl group, as defined herein.

A "thioaryloxy" group refers to both an -S-aryl and an -S-heteroaryl group, as defined herein.

A "thioether" refers to both a thioalkoxy and a thioaryloxy group, wherein the group is linked to an alkyl, alkenyl, alkynyl, cycloalkyl, aryl, heteroaryl or heteroalicyclic group.

A thioether bond describes a -S- bond.

A "disulfide" group refers to both a -S-thioalkoxy and a -S-thioaryloxy group. A disulfide bond describes a -S-S- bond.

A "carbonyl" group refers to a -C(=O)-R' group, where R' is defined as hereinabove.

A "thiocarbonyl" group refers to a -C(=S)-R' group, where R' is as defined herein.

A "carboxyl" refers to both "C-carboxy" and O-carboxy".

A "C-carboxy" group refers to a -C(=O)-O-R' groups, where R' is as defined herein.

An "O-carboxy" group refers to an R'C(=O)-O- group, where R' is as defined herein.

An "oxo" group refers to a =O group.

A "carboxylate" or "carboxyl" encompasses both C-carboxy and O-carboxy groups, as defined herein.

A "carboxylic acid" group refers to a C-carboxy group in which R' is hydrogen.

A "thiocarboxy" or "thiocarboxylate" group refers to both -C(=S)-O-R' and -O-C(=S)R' groups.

An "ester" refers to a C-carboxy group wherein R' is not hydrogen.

An ester bond refers to a -O-C(=O)- bond.

A thioester bond refers to a -0-C(=S)- bond or to a -S-C(=O) bond.

A "halo" group refers to fluorine, chlorine, bromine or iodine.

A "sulfinyl" group refers to an -S(=O)-R' group, where R' is as defined herein.

A "sulfonyl" group refers to an -S(=O)₂-R' group, where R' is as defined herein.

A "sulfonate" group refers to an -S(=O)₂-O-R' group, where R' is as defined herein.

A "sulfate" group refers to an -O-S(=O)₂-O-R' group, where R' is as defined as herein.

A "sulfonamide" or "sulfonamido" group encompasses both S-sulfonamido and N-sulfonamido groups, as defined herein.

An "S-sulfonamido" group refers to a -S(=O)₂-NR'R'' group, with each of R' and R" as defined herein.

An "N-sulfonamido" group refers to an R'S(=O)₂-NR'' group, where each of R' and R'' is as defined herein.

An "O-carbamyl" group refers to an -OC(=O)-NR'R" group, where each of R' and R" is as defined herein.

An "N-carbamyl" group refers to an R'OC(=O)-NR"- group, where each of R' and R" is as defined herein.

A "carbamyl" or "carbamate" group encompasses O-carbamyl and N-carbamyl groups.

A carbamate bond describes a -O-C(=O)-NR'- bond, where R' is as described herein.

An "O-thiocarbamyl" group refers to an -OC(=S)-NR'R" group, where each of R' and R'' is as defined herein.

An "N-thiocarbamyl" group refers to an R'OC(=S)NR"- group, where each of R' and R'' is as defined herein.

A "thiocarbamyl" or "thiocarbamate" group encompasses O-thiocarbamyl and N-thiocarbamyl groups.

A thiocarbamate bond describes a -O-C(=S)-NR'- bond, where R' is as described herein.

A "C-amido" group refers to a -C(=O)-NR'R" group, where each of R' and R'' is as defined herein.

An "N-amido" group refers to an R'C(=O)-NR"- group, where each of R' and R" is as defined herein.

An "amide" group encompasses both C-amido and N-amido groups.

An amide bond describes a -NR'-C(=O)- bond, where R' is as defined herein.

An amine bond describes a bond between a nitrogen atom in an amine group (as defined herein) and an R' group in the amine group.

A thioamide bond describes a -NR'-C(=S)- bond, where R' is as defined herein.

A "urea" group refers to an N(R')-C(=O)-NR"R"' group, where each of R' and R" is as defined herein, and R"' is defined as R' and R'' are defined herein.

A "nitro" group refers to an -NO₂ group.

A "cyano" group refers to a -C≡N group.

The term "phosphonyl" or "phosphonate" describes a -P(=O)(OR')(OR") group, with R' and R'' as defined hereinabove.

The term "phosphate" describes an -O-P(=O)(OR')(OR") group, with each of R' and R'' as defined hereinabove.

A "phosphoric acid" is a phosphate group is which each of R is hydrogen.

The term "phosphinyl" describes a -PR'R" group, with each of R' and R'' as defined hereinabove.

The term "thiourea" describes a -N(R')-C(=S)-NR''- group, with each of R' and R" as defined hereinabove.

As described herein, multimeric protein structures described herein may exhibit longer lasting glucocerebrosidase activity at therapeutically important sites *in vivo.* Such multimeric protein structures are therefore highly beneficial for use in various medical applications in which glucocerebrosidase activity is desirable, including therapeutic and research applications.

Hence, according to some embodiments, the multimeric protein structure described herein is for use as a medicament, for example, a medicament for treating Gaucher disease.

According to another aspect of embodiments of the invention, there is provided a method of treating Gaucher disease, the method comprising administering to a subject in need thereof a therapeutically effective amount of a multimeric protein structure described herein.

In any of the methods and uses described herein, the multimeric structures of GCD as described herein can be utilized either *per se,* or, preferably, as a part of a pharmaceutical composition with further comprises a pharmaceutically acceptable carrier.

According to another aspect of embodiments of the invention, there is provided a pharmaceutical composition that comprises a multimeric protein structure as described herein and a pharmaceutically acceptable carrier.

As used herein a "pharmaceutical composition" refers to a preparation of one or more of the multimeric protein structures described herein, with other chemical components such as pharmaceutically acceptable and suitable carriers and excipients. The purpose of a pharmaceutical composition is to facilitate administration of a compound to an organism.

Hereinafter, the term "pharmaceutically acceptable carrier" refers to a carrier or a diluent that does not cause significant irritation to an organism and does not abrogate the biological activity and properties of the administered compound. Examples, without limitations, of carriers are: propylene glycol, saline, emulsions and mixtures of organic solvents with water, as well as solid (e.g., powdered) and gaseous carriers.

Herein the term "excipient" refers to an inert substance added to a pharmaceutical composition to further facilitate administration of a compound. Examples, without limitation, of excipients include calcium carbonate, calcium phosphate, various sugars and types of starch, cellulose derivatives, gelatin, vegetable oils and polyethylene glycols.

In some embodiments, the pharmaceutical composition further comprises an additional ingredient selected from the group consisting of glucose, a saccharide comprising a glucose moiety, nojirimycin, and derivatives thereof. The saccharide may be a disaccharide comprising at least one glucose moiety, a trisaccharide comprising at least one glucose moiety, or an oligosaccharide or polysaccharide comprising at least one glucose moiety. In some embodiments, the saccharide is a disaccharide, and in some embodiments, the saccharide is sucrose.

Techniques for formulation and administration of drugs may be found in "Remington's Pharmaceutical Sciences" Mack Publishing Co., Easton, PA, latest edition.

Pharmaceutical compositions of the present invention may be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with the present invention thus may be formulated in conventional manner using one or more pharmaceutically acceptable carriers comprising excipients and auxiliaries, which facilitate processing of the multimeric protein structure into preparations which can be used pharmaceutically. Proper formulation is dependent upon the route of administration chosen.

For injection or infusion, the multimeric protein structures of embodiments of the invention may be formulated in aqueous solutions, preferably in physiologically compatible buffers such as Hank's solution, Ringer's solution, or physiological saline buffer with or without organic solvents such as propylene glycol, polyethylene glycol.

For transmucosal administration, penetrants are used in the formulation. Such penetrants are generally known in the art.

For oral administration, the multimeric protein structures of the invention can be formulated readily by combining the multimeric protein structures with pharmaceutically acceptable carriers well known in the art. Such carriers enable the multimeric protein structures described herein to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethylcellulose, sodium carbomethylcellulose; and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of doses of active multimeric protein structure.

Pharmaceutical compositions, which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the multimeric protein structures may be dissolved or suspended in suitable liquids. In addition, stabilizers may be added. All formulations for oral administration should be in dosages suitable for the chosen route of administration.

For buccal administration, the compositions may take the form of tablets or lozenges formulated in conventional manner.

For administration by inhalation, the multimeric protein structures for use according to embodiments of the present invention are conveniently delivered in the form of an aerosol spray presentation (which typically includes powdered, liquified and/or gaseous carriers) from a pressurized pack or a nebulizer, with the use of a suitable propellant, e.g., dichlorodifluoromethane, trichlorofluoromethane, dichlorotetrafluoroethane or carbon dioxide. In the case of a pressurized aerosol, the dosage unit may be determined by providing a valve to deliver a metered amount. Capsules and cartridges of, e.g., gelatin for use in an inhaler or insufflator may be formulated containing a powder mix of the multimeric protein structures and a suitable powder base such as, but not limited to, lactose or starch.

The multimeric protein structures described herein may be formulated for parenteral administration, e.g., by bolus injection or continuous infusion. Formulations for injection or infusion may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous solutions of the multimeric protein structure preparation in water-soluble form. Additionally, suspensions of the multimeric protein structures may be prepared as appropriate oily injection suspensions and emulsions (e.g., water-in-oil, oil-in-water or water-in-oil in oil emulsions). Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents, which increase the solubility of the multimeric protein structures to allow for the preparation of highly concentrated solutions.

Alternatively, the multimeric protein structures may be in powder form for constitution with a suitable vehicle, e.g., sterile, pyrogen-free water, before use.

The multimeric protein structure of embodiments of the present invention may also be formulated in rectal compositions such as suppositories or retention enemas, using, e.g., conventional suppository bases such as cocoa butter or other glycerides.

The pharmaceutical compositions herein described may also comprise suitable solid of gel phase carriers or excipients. Examples of such carriers or excipients include, but are not limited to, calcium carbonate, calcium phosphate, various sugars, starches, cellulose derivatives, gelatin and polymers such as polyethylene glycols.

Pharmaceutical compositions suitable for use in the context of the present invention include compositions wherein the active ingredients are contained in an amount effective to achieve the intended purpose. More specifically, a therapeutically effective amount means an amount of multimeric protein structures effective to prevent, alleviate or ameliorate symptoms of disease or prolong the survival of the subject being treated.

For any multimeric protein structures used in the methods of the invention, the therapeutically effective amount or dose can be estimated initially from activity assays in animals. For example, a dose can be formulated in animal models to achieve a circulating concentration range that includes the IC₅₀ as determined by activity assays (e.g., the concentration of the test protein structures, which achieves a half-maximal increase in a biological activity of the multimeric protein structure). Such information can be used to more accurately determine useful doses in humans.

As is demonstrated in the Examples section that follows, a therapeutically effective amount for the multimeric protein structures of embodiments of the present invention may range between about 1 µg/kg body weight and about 500 mg/kg body weight.

Toxicity and therapeutic efficacy of the multimeric protein structures described herein can be determined by standard pharmaceutical procedures in experimental animals, e.g., by determining the EC₅₀, the IC₅₀ and the LD₅₀ (lethal dose causing death in 50 % of the tested animals) for a subject protein structure. The data obtained from these activity assays and animal studies can be used in formulating a range of dosage for use in human.

The dosage may vary depending upon the dosage form employed and the route of administration utilized. The exact formulation, route of administration and dosage can be chosen by the individual physician in view of the patient's condition. (See e.g., Fingl et al., 1975, in "The Pharmacological Basis of Therapeutics", Ch. 1 p.1).

Dosage amount and interval may be adjusted individually to provide plasma levels of the active moiety which are sufficient to maintain the desired effects, termed the minimal effective concentration (MEC). The MEC will vary for each preparation but can be estimated from *in vitro* data; e.g., the concentration necessary to achieve the desired level of activity *in vitro.* Dosages necessary to achieve the MEC will depend on individual characteristics and route of administration. HPLC assays or bioassays can be used to determine plasma concentrations.

Dosage intervals can also be determined using the MEC value. Preparations should be administered using a regimen, which maintains plasma levels above the MEC for 10-90 % of the time, preferably between 30-90 % and most preferably 50-90 %.

The amount of a composition to be administered will, of course, be dependent on the subject being treated, the severity of the affliction, the manner of administration, the judgment of the prescribing physician, etc.

Compositions of the present invention may, if desired, be presented in a pack or dispenser device, such as an FDA (the U.S. Food and Drug Administration) approved kit, which may contain one or more unit dosage forms containing the active ingredient. The pack may, for example, comprise metal or plastic foil, such as, but not limited to a blister pack or a pressurized container (for inhalation). The pack or dispenser device may be accompanied by instructions for administration. The pack or dispenser may also be accompanied by a notice associated with the container in a form prescribed by a governmental agency regulating the manufacture, use or sale of pharmaceuticals, which notice is reflective of approval by the agency of the form of the compositions for human or veterinary administration. Such notice, for example, may be of labeling approved by the U.S. Food and Drug Administration for prescription drugs or of an approved product insert. Compositions comprising a multimeric protein structure of embodiments of the invention formulated in a compatible pharmaceutical carrier may also be prepared, placed in an appropriate container, and labeled for treatment of an indicated condition or diagnosis, as is detailed herein.

Thus, according to an embodiment of the present invention, depending on the selected multimeric protein structures, the pharmaceutical composition described herein is packaged in a packaging material and identified in print, in or on the packaging material, for use in the treatment of a condition in which the activity of the multimeric protein structure is beneficial, as described hereinabove.

As used herein the term "about" refers to ± 10 %

The terms "comprises", "comprising", "includes", "including", "having" and their conjugates mean "including but not limited to".

The term "consisting of means "including and limited to".

The word "exemplary" is used herein to mean "serving as an example, instance or illustration". Any embodiment described as "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the invention may include a plurality of "optional" features unless such features conflict.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4; from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non-limiting fashion.

### MATERIALS AND METHODS

### Materials:

Monofunctional and bi-functional PEGs (bis-NHS-PEGs and MeO-PEG-NHS) were obtained from commercially vendors such as Sigma-Aldrich, NOF corporation, Quanta Biodesign, LAYSAN bio inc., Nanocs Inc., SunBio Inc., JenKem Technology, Rapp Polymere, IRIS Biotech GmbH and CreativePEGWorks. Bis-NHS-PEG₅ (PEG1435, MW=532.51) was obtained from IRIS Biotech GmbH;
Citric acid was obtained from Sigma;
Coomassie Blue G250 was obtained from Bio-Rad;
Dimethyl sulfoxide (DMSO) was obtained from Sigma;
Glycine was obtained from Sigma;
Human plasma (K3 EDTA) was obtained from Bioreclamation Inc.;
Mannan was obtained from Sigma;
4-Methylumbelliferone was obtained from Sigma;
4-Methylumbelliferyl-β-D-glucopyranoside was obtained from Sigma;
Phosphate buffered saline (PBS) was obtained from Sigma;
*p*-Nitrophenyl-β-D-glucopyranoside was obtained from Sigma;
Sinapinic acid was obtained from Sigma;
Sodium hydroxide was obtained from Sigma;
Sodium phosphate was obtained from Sigma;
Sodium taurocholate was obtained from Sigma;
Trifluoroacetic acid was obtained from Sigma.

Glucocerebrosidase having SEQ ID NO: 3, and being characterized by a terminal mannose content, was prepared as described in International Patent Applications PCT/IL2004/000181 (published as WO 2004/096978) and PCT/IL2008/000756 (published as WO 2008/132743).

### Macrophage cell line:

NR8383 rat alveolar macrophages were obtained from American Type Culture Collection (CRL-2192 cells).

### SDS-PAGE:

SDS-PAGE was carried out under reducing conditions using an Invitrogen Novex® mini-cell and precasted NuPAGE® Novex 3-8 % Tris-Acetate Gel 1.5 mm. The gel was stained by Coomassie Blue G250 stain.

### IEF (isoelectric focusing):

IEF was carried out using an Invitrogen Novex® mini-cell and precasted IEF gels having a pH range of 3-7 (Invitrogen). The gel was stained by Coomassie Blue G250.

### Mass spectrometry (MALDI-TOF):

MALDI-TOF was performed using a Bruker Reflex IV MALDI-ToF Mass-spectrometer system (Bruker-Franzen Analytik GmbH, Germany) and a sinapinic acid/trifluoroacetic acid (TFA) (0.1 % TFA/acetonitrile (2:1, v/v)) saturated matrix solution.

### GCD activity assays:

Kinetic parameters for enzymatic activity were determined by measuring hydrolysis of synthetic substrates, either *p*-nitrophenyl-β-D-glucopyranoside (pNP-G), or 4-methylumbelliferyl-β-D-glucopyranoside (4MU-G). In both cases, the reaction was initiated by addition of 10 µL of the tested enzyme to an activity buffer (pH 5.5), at a final concentration of 0.1 µg/mL. The reaction was then maintained for 45 minutes at a temperature of 37 °C.

For pNP-G assays, the activity buffer contained 20 mM citrate, 30 mM sodium phosphate, 0.125 % taurocholic acid, and 1.5 % Triton X-100. The reaction was quenched at the end of 45 minutes with 20 mL of aqueous sodium hydroxide (5 N), and the absorbance of the alkaline solutions containing the phenolate product was measured at a wavelength of 405 nm, using a Varian Cary^{®} 50 spectrometer (Agilent Technologies). The concentration of phenolate was determined quantitatively based on an appropriate calibration curve.

For 4MU-G assays, the activity buffer contained 50 mM citrate, 176 mM potassium phosphate, 10 mM taurocholic acid, and 0.01 % Tween 20. At the end of 45 minutes, 10 µl samples of the reaction mixture were added to 90 µl of stop solution (1 M sodium hydroxide, 1 M glycine, pH 10), and the fluorescence was measured at excitation/emission wavelengths of 370/440 nm, using an Infinite^{®} M200 fluorometer (Tecan). The concentration of the 4-methylumbelliferone (4MU) product was obtained quantitatively based on an appropriate calibration curve.

### EXAMPLE 1

### Cross-linking of glucocerebrosidase (GCD) with bis-N-hydroxysuccinimide-poly(ethylene glycol) (bis-NHS-PEG)

Plant recombinant human GCD (prh-GCD) was cross-linked with bis-*N-*hydroxysuccinimide-poly(ethylene glycol) (bis-NHS-PEG) at 50:1 and 100:1 molar ratios of bis-NHS-PEG to GCD. In order to investigate the effect of cross-linker length on the cross-linking reaction, bis-NHS-PEG with various lengths of poly(ethylene glycol) (PEG) chains were used: bis-NHS-PEG₅, bis-NHS-PEG₈, bis-NHS-PEG₂₁ (bis-NHS-PEG with a 1 KDa PEG chain), bis-NHS-PEG₄₅ (bis-NHS-PEG with a 2 KDa PEG chain), bis-NHS-PEG₆₈ (bis-NHS-PEG with 3 KDa PEG), and bis-NHS-PEG₁₃₆ (bis-NHS-PEG with 6 KDa PEG).

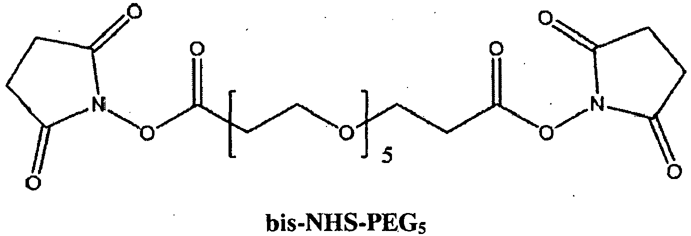

Fresh stock solutions of bis-NHS-PEG in DMSO were prepared at the following concentrations: bis-NHS-PEG₅ 4.4 mg/ml; bis-NHS-PEG₈ 5.8 mg/ml; bis-NHS-PEG₂₁ 10.2 mg/ml; bis-NHS-PEG₄₅ 16 mg/ml; bis-NHS-PEG₆₈ 25.8 mg/ml; bis-NHS-PEG₁₃₆ 56.5 mg/ml.

For cross-linking with a 50:1 molar excess of reagent, 10 µL of bis-NHS-PEG stock solution was added (optionally dropwise) to 90 µL of phosphate buffer (100 mM, pH 8) containing 100 µg of prh-GCD and 100 mg/ml sucrose. For cross-linking with a 100:1 molar excess of reagent, 20 µL of the bis-NHS-PEG stock solution was added to 80 µL of phosphate buffer (100 mM, pH 8) containing 100 µg of prh-GCD and 100 mg/ml sucrose. The cross-linking reaction mixture is gently agitated for 2 hours at room temperature and then dialyzed against an appropriate solution (e.g. saline, appropriate buffer) using a membrane with a nominal molecular weight cut-off of 50 KDa.

The cross-linking was then analyzed by SDS-PAGE analysis.

As shown in Figure 1, in each sample of GCD reacted with bis-NHS-PEG, the GCD exhibited a shift to higher molecular weights, indicating the presence of a covalent dimer. As further shown therein, reacting GCD with bis-NHS-PEG₅ and bis-NHS-PEG₈ resulted in a larger proportion of dimeric GCD than did reacting GCD with longer bis-NHS-PEG reagents.

As is further shown therein, the molecular weight of the monomeric portion of GCD increased following reaction with the cross-linker, indicating that protein monomers which were not dimerized by cross-linking, were covalently attached to the bis-NHS-PEG cross-linker, i.e., the proteins were PEGylated.

This result indicates that bis-NHS-PEG₅ and bis-NHS-PEG₈ are more synthetically efficient cross-linkers of GCD than are longer bis-NHS-PEG reagents.

In order to better characterize the effect of the molar ratio of cross-linking reagent to GCD, prh-GCD was cross-linked with bis-NHS-PEG₅ at 25:1, 50:1, 75:1, 100:1 and 200:1 bis-NHS-PEG₅:GCD molar ratios, using procedures similar to those described hereinabove.

125, 187, 250, or 500 µL of a solution of 33 mg/ml bis-NHS-PEG₅ in DMSO was added to 9.9 mL of phosphate buffer (100 mM, pH 8) containing 10 mg of prh-GCD and 100 mg/ml sucrose, in order to obtain molar ratios 50:1, 75:1, 100:1 and 200:1. To obtain a molar ratio of 25:1, 100 µL of a solution of 20 mg/ml bis-NHS-PEG₅ in DMSO was added. The reaction mixtures were agitated for 2 hours at room temperature and then dialyzed against an appropriate solution (e.g. saline, appropriate buffer) using a membrane with a nominal molecular weight cut-off of 50 KDa.

The cross-linking reactions were analyzed by SDS-PAGE, IEF (isoelectric focusing), MALDI-TOF mass spectrometry, and 4-methylumbelliferyl-β-D-glucopyranoside activity assays, as described in the Materials and Methods section hereinabove.

As shown in Table 1 below, cross-linking of prh-GCD resulted in some loss of enzymatic activity, yet it is suggested that the loss of activity merely results from the reaction conditions used and that it may be obviated upon optimization, and hence should not be regarded as indicative of the multimeric structure as described herein.

Optimizing the conditions used for preparing a multimeric structure of GCD as described herein include, for example, evaluating the effect of the reaction temperature and/or pH, the solvent composition, the buffer used, the ionic strength of the reaction's solution, the use of isotonicity modifiers, the use of reversible inhibitors to protect the active site of the protein (e.g., nojirimycin derivatives), the use of different excipients, the use of surface active molecules (e.g., TWEEN), and of the reaction duration.

Additional optimization may include purification of the active CL-GCD using standard chromatography techniques or/and affinity-based chromatography (e.g., using reversible inhibitors as affinity ligands).

**Table 1: Activity of prh-GCD cross-linked with bis-NHS-PEG₅**

| **Molar ratio of bis-NHS-PEG₅ : GCD** | **Activity of cross-linked GCD relative to activity of non-cross-linked GCD** |
|---|---|
| 25:1 | 63 % |
| 75:1 | 59 % |
| 200:1 | 44 % |

As shown in Figure 2, prh-GCD was primarily in a dimeric form for each of the tested molar ratios, but molar ratios of 75:1, 100:1 and 200:1 bis-NHS-PEG₅: GCD each provided particularly high percentages of the dimer.

As further shown therein, cross-linking increased the molecular weight of prh-GCD monomers to a degree which was correlated to the bis-NHS-PEG₅:GCD molar ratio.

As shown in Figure 3, cross-linking decreased the isoelectric point of prh-GCD considerably, to a degree which was correlated to the bis-NHS-PEG₅: GCD molar ratio.

As shown in Figures 4A and 4B, reacting prh-GCD with a 75:1 molar excess of bis-NHS-PEG₅ increased the molecular weight of the prh-GCD monomer from 60.8 KDa to 64.2 KDa (indicating modification with about 11 reagent molecules) and of the prh-GCD dimer from 121.4 KDa to 124.0 KDa (indicating modification with about 7.4 reagent molecules).

As shown in Table 2, the degree of modifications to the prh-GCD, as determined by MALDI-TOF mass spectrometry, was correlated to the bis-NHS₅: GCD molar ratio.

**Table 2: Number of modifications to prh-GCD cross-linked with bis-NHS-PEG₅**

| **Molar ratio of bis-NHS-PEG₅: GCD** | **PEG moieties per prh-GCD dimer** |
|---|---|
| 50:1 | 4.3 |
| 75:1 | 7.4 |
| 100:1 | 8.4 |
| 200:1 | 12.5 |

The above results indicate that higher bis-NHS-PEG: GCD molar ratios result in a greater number of PEG cross-linking moieties attached to the GCD via amide bonds, and consequently an increased molecular weight and fewer free amine groups.

### EXAMPLE 2

### Effect of cross-linking on stability of glucocerebrosidase (GCD) in solution

The activity of cross-linked plant recombinant human GCD (prh-GCD) was determined in plasma and in simulated lysosomal conditions, in order to assess the stability of the cross-linked prh-GCD under these conditions. For comparison, the activities of non-modified prh-GCD and of non-cross-linked PEGylated prh-GCD were also determined.

Cross-linked prh-GCD was prepared by reacting prh-GCD with bis-NHS-PEG₅ at a 1:25, 1:75 or 1:200 molar ratio, as described in Example 1.

Non-cross-linked PEGylated prh-GCD was prepared by reacting prh-GCD with by methoxy-capped PEG₈-NHS (MeO-PEG₈-NHS) at a 50:1 molar ratio. 3.98 mg of MeO-PEG₈-NHS in 45 µl DMSO was added from a freshly prepared DMSO stock solution to 9 mL of phosphate buffer (100 mM, pH 8) containing 10 mg of prh-GCD and 100 mg/ml sucrose. The reaction mixture was gently agitated for 2 hours at room temperature and then dialyzed against an appropriate solution (e.g. saline, appropriate buffer) using a membrane with a nominal molecular weight cut-off of 50 KDa.

As shown in Figures 5 and 6, cross-linked prh-GCD exhibited a considerably longer lasting activity in both plasma (Figure 5) and simulated lysosomal conditions (Figure 6), in comparison to both non-modified prh-GCD and non-cross-linked PEGylated prh-GCD. As further shown therein, the GCD was noticeably more stabilized by cross-linking with 75:1 and 200:1 molar ratios than by cross-linking with a 25:1 molar ratio.

As further shown in Figure 5, the activity of non-cross-linked PEGylated prh-GCD decayed in a manner very similar to that of non-modified prh-GCD. This indicates that PEGylation *per se* (i.e., without the effects of cross-linking) has little if any effect on the stability of GCD under the tested conditions.

The above results indicate that formation of a covalent dimer by cross-linking generates longer lasting GCD activity.

### EXAMPLE 3

### Effect of cross-linking on uptake and stability of glucocerebrosidase (GCD) in macrophages in vitro

The cellular uptake of cross-linked GCD was determined using rat alveolar macrophages.

Crosslinked prh-GCD was prepared by reacting prh-GCD with bis-NHS-PEG₅ at a 50:1 bis-NHS-PEG₅:GCD molar ratio, as described in Example 1.

Rat alveolar macrophages were placed in wells of 96-well plates at a concentration of 0.5-1 x 10⁶ cells in 200 µL medium per well (6 wells for each treatment group). 25 µL of a 300 µg/mL solution of prh-GCD or cross-linked prh-GCD was added to each well, along with 25 µL of water or a 10 mg/ml solution of mannan (an inhibitor of uptake via the mannose receptor). The samples were then incubated for two hours at 37 °C in an atmosphere with 5 % CO₂.

After incubation, the cells were washed twice in PBS (phosphate buffered solution) with 1 mg/mL mannan, and then twice with PBS, in order to remove remaining GCD. The cells were harvested and lysed with 100 µL lysis buffer (60 mM phosphate citrate buffer, 0.15 % Triton X-100, 0.125 % sodium taurocholate, pH 5.5) and one freeze-thaw cycle and pipettation. GCD activity in the cell lysate was determined by a *p*-nitrophenyl-β-D-glucopyranoside activity assay, as described hereinabove.

In the absence of mannan, the uptake of cross-linked prh-GCD (200 ng/mL lysate was similar to, but slightly lower than that of non-cross-linked prh-GCD (333 ng/mL lysate).

The lower value obtained for cross-linked prh-GCD may be due to a presence of inactive enzyme in the cross-linked prh-GCD (in accordance with the results presented in Table 1 hereinabove), which undergoes uptake but is not detected by the activity assay.

Mannan reduced the uptake of cross-linked prh-GCD by 85 % and the uptake of non-cross-linked prh-GCD by 90 %, indicating that both proteins undergo uptake via mannose receptor.

The above results indicate that cross-linking of GCD has little or no negative effect on the uptake of GCD by cells and that the mechanism of GCD uptake was unchanged by the cross-linking.

### EXAMPLE 4

### Effect of cross-linking on biodistribution and stability of glucocerebrosidase (GCD) in vivo

The biodistribution of non-cross-linked prh-GCD and of prh-GCD cross-linked with bis-NHS-PEG₅ were determined for comparison.

Mice were injected with prh-GCD cross-linked by bis-NHS-PEG₅ (prepared as described in Example 1 using 75 molar equivalents of bis-NHS-PEG₅) or with non-cross-linked prh-GCD, at a dose of 5 mg/Kg GCD (as determined by activity assay). Blood samples and organs (liver and spleen) were collected 1, 4, 8, 24, 48, and 72 hours post-injection. Each treatment/time point group consisted of six male ICR mice. Organs were lysed with extraction buffer (20 mM phosphate buffer pH 7.2, 20 mM EDTA, 20 mM L-ascorbic acid, 1 % Triton X-100) at a tissue:buffer weight ratio of 1:5. The GCD activity in plasma, liver and spleen was determined by 4-methylumbelliferyl-β-D-glucopyranoside activity assays, as described in the Materials and Methods section hereinabove.

As shown in Figure 7A, cross-linked prh-GCD exhibited a considerably greater presence in plasma than did non-cross-linked prh-GCD following injection. One hour after injection, the non-cross-linked prh-GCD was almost completely eliminated from blood circulation, whereas the cross-linked prh-GCD exhibited approximately ten times as much activity as did the non-cross-linked prh-GCD.

Similarly, as shown in Figures 7B and 7C, cross-linked prh-GCD exhibited a considerably greater activity in liver (Figure 7B) and spleen (Figure 7C) than did non-cross-linked prh-GCD following injection.

These results indicate that cross-linking GCD can lead to considerably higher levels of GCD activity *in vivo,* in the circulation and in target organs.

Although the invention has been described in conjunction with specific embodiments thereof, it is evident that many alternatives, modifications and variations will be apparent to those skilled in the art. Citation or identification of any reference in this application shall not be construed as an admission that such reference is available as prior art to the present invention. To the extent that section headings are used, they should not be construed as necessarily limiting.

## Claims

1. A multimeric protein structure comprising at least two glucocerebrosidase molecules being covalently linked to one another via a linking moiety, the multimeric protein structure featuring a characteristic selected from the group consisting of:
(a) a glucocerebrosidase activity upon subjecting the multimeric protein structure to human plasma conditions for one hour, which is at least 10 % higher than an activity of native glucocerebrosidase upon subjecting said native glucocerebrosidase to said human plasma conditions for one hour;
(b) a glucocerebrosidase activity which decreases upon subjecting the multimeric protein structure to human plasma conditions for one hour by a percentage which is at least 10 % less than the percentage by which an activity of said native glucocerebrosidase decreases upon subjecting said native glucocerebrosidase to said human plasma conditions for one hour;
(c) a glucocerebrosidase activity which remains substantially unchanged upon subjecting the multimeric protein structure to human plasma conditions for one hour;
(d) a glucocerebrosidase activity, upon subjecting the multimeric protein structure to lysosomal conditions for 4 days, which is at least 10 % higher than an activity of native glucocerebrosidase upon subjecting said native glucocerebrosidase to said lysosomal conditions for 4 days;
(e) a glucocerebrosidase activity which decreases upon subjecting the multimeric protein structure to lysosomal conditions for one day by a percentage which is at least 10 % less than the percentage by which an activity of said native glucocerebrosidase decreases upon subjecting said native glucocerebrosidase to said lysosomal conditions for one day;
(f) a glucocerebrosidase activity which remains substantially unchanged upon subjecting the multimeric protein structure to lysosomal conditions for one day; and
(g) a circulating half-life in a physiological system which is higher by at least 20 % than a circulating half-life of said native glucocerebrosidase.

2. A multimeric protein structure comprising at least two glucocerebrosidase molecules being covalently linked to one another via a linking moiety, wherein said linking moiety is not present in native glucocerebrosidase.

3. The multimeric protein structure of claim 2, featuring a characteristic selected from the group consisting of:
(a) a glucocerebrosidase activity upon subjecting the multimeric protein structure to human plasma conditions for one hour, which is at least 10 % higher than an activity of native glucocerebrosidase upon subjecting said native glucocerebrosidase to said human plasma conditions for one hour;
(b) a glucocerebrosidase activity which decreases upon subjecting the multimeric protein structure to human plasma conditions for one hour by a percentage which is at least 10 % less than the percentage by which an activity of said native glucocerebrosidase decreases upon subjecting said native glucocerebrosidase to said human plasma conditions for one hour;
(c) a glucocerebrosidase activity which remains substantially unchanged upon subjecting the multimeric protein structure to human plasma conditions for one hour;
(d) a glucocerebrosidase activity, upon subjecting the multimeric protein structure to lysosomal conditions for 4 days, which is at least 10 % higher than an activity of native glucocerebrosidase upon subjecting said native glucocerebrosidase to said lysosomal conditions for 4 days;
(e) a glucocerebrosidase activity which decreases upon subjecting the multimeric protein structure to lysosomal conditions for one day by a percentage which is at least 10 % less than the percentage by which an activity of said native glucocerebrosidase decreases upon subjecting said native glucocerebrosidase to said lysosomal conditions for one day;
(f) a glucocerebrosidase activity which remains substantially unchanged upon subjecting the multimeric protein structure to lysosomal conditions for one day; and
(g) a circulating half-life in a physiological system which is higher by at least 20 % than a circulating half-life of said native glucocerebrosidase.

4. The multimeric protein structure of any of claims 1 and 3, wherein said circulating half-life of the multimeric protein structure which is higher than a circulating half-life of said native glucocerebrosidase, is higher by at least 50 % than said circulating half-life of said native glucocerebrosidase.

5. The multimeric protein structure of any of claims 1, 3 and 4, being **characterized by** a glucocerebrosidase activity upon subjecting the multimeric protein structure to human plasma conditions for one hour, which is at least 10-fold an activity of native glucocerebrosidase upon subjecting said native glucocerebrosidase to said human plasma conditions for one hour.

6. The multimeric protein structure of any of claims 1 to 5, **characterized by** a glucocerebrosidase activity in an organ upon administration of said multimeric protein structure to a vertebrate, said organ being selected from the group consisting of a liver, a spleen, a kidney, a lung, a bone marrow and blood.

7. The multimeric protein structure of any of claims 1 to 6, comprising two glucocerebrosidase molecules, the protein structure being a dimeric protein structure.

8. The multimeric protein structure of any of claims 1 to 7, wherein said glucocerebrosidase has an amino acids sequence selected from the group consisting of SEQ ID NO: 1, SEQ ID NO: 2, and SEQ ID NO: 3.

9. The multimeric protein structure of any of claims 1 to 8, wherein said linking moiety comprises a poly(alkylene glycol).

10. The multimeric protein structure of any of claims 1 to 8, wherein said at least one linking moiety has a general formula:
-X₁-(CR₁R₂-CR₃R₄-Y)n-X₂-
wherein each of X₁ and X₂ is a functional group that forms a covalent bond with at least one glucocerebrosidase molecule;
Y is O, S or NR₅;
n is an integer from 1 to 200; and
each of R₁, R₂, R₃, R₄ and R₅ is independently selected from the group consisting of hydrogen, alkyl, cycloalkyl, alkenyl, alkynyl, alkoxy, hydroxy, oxo, thiol and thioalkoxy.

11. A pharmaceutical composition comprising the multimeric protein structure of any of claims 1 to 10 and a pharmaceutically acceptable carrier.

12. The pharmaceutical composition of claim 11, further comprising an ingredient selected from the group consisting of glucose, a saccharide comprising a glucose moiety, nojirimycin, and derivatives thereof.

13. The multimeric protein structure of any of claims 1 to 10, for use in treating Gaucher disease.

14. A process of preparing the multimeric protein structure of any of claims 1 to 10, the process comprising reacting glucocerebrosidase with a cross-linking agent which comprises said linking moiety and at least two reactive groups.

15. The process of claim 14, wherein a molar ratio of said cross-linking agent to said glucocerebrosidase is in a range of from 5:1 to 500:1.

## Patentansprüche

1. Multimere Proteinstruktur, die wenigstens zwei Glucocerebrosidase-Moleküle umfaßt, die über eine Verbindungs-Einheit kovalent aneinander gebunden sind, wobei die multimere Proteinstruktur ein Merkmal aufweist, das ausgewählt ist aus der Gruppe, die besteht aus:
(a) einer Glucocerebrosidase-Aktivität, nachdem die multimere Proteinstruktur Humanplasma-Bedingungen für eine Stunde unterworfen wurde, die wenigstens 10 % höher ist als eine Aktivität von nativer Glucocerebrosidase, nachdem die native Glucocerebrosidase den Humanplasma-Bedingungen für eine Stunde unterworfen wurde;
(b) einer Glucocerebrosidase-Aktivität, die sich verringert, nachdem die mulimere Proteinstruktur Humanplasma-Bedingungen für eine Stunde unterworfen wurde, um einen Prozentsatz, der wenigstens 10 % weniger ist als der Prozentsatz, um den sich eine Aktivität der nativen Glucocerebrosidase verringert, nachdem die native Glucocerebrosidase den Humanplasma-Bedingungen für eine Stunde unterworfen wurde;
(c) einer Glucocerebrosidase-Aktivität, die im wesentlich unverändert bleibt, nachdem die multimere Proteinstruktur Humanplasma-Bedingungen für eine Stunde unterworfen wurde;
(d) einer Glucocerebrosidase-Aktivität, nachdem die multimere Proteinstruktur lysosomalen Bedingungen für 4 Tage unterworfen wurde, die wenigstens 10 % höher ist als eine Aktivität von nativer Glucocerebrosidase, nachdem die native Glucocerebrosidase den lysosomalen Bedingungen für 4 Tage unterworfen wurde.
(e) einer Glucocerebrosidase-Aktivität, die sich verringert, wenn die multimere Proteinstruktur lysosomalen Bedingungen für einen Tag unterworfen wurde, um einen Prozentsatz, der wenigstens 10 % weniger ist als der Prozentsatz, um den sich eine Aktivität der nativen Glucocerebrosidase verringert, nachdem die native Glucocerebrosidase den lysosomalen Bedingungen für einen Tag unterworfen wurde;
(f) einer Glucocerebrosidase-Aktivität, die im wesentlichen unverändert bleibt, nachdem die multimere Proteinstruktur lysosomalen Bedingungen für einen Tag unterworfen wurde; und
(g) einer zirkulierenden Halbwertszeit in einem physiologischen System, die um wenigstens 20 % höher ist als eine zirkulierende Halbwertszeit der nativen Glucocerebrosidase.

2. Multimere Proteinstruktur, die wenigstens zwei Glucocerebrosidase-Moleküle umfaßt, die über eine Verbindungs-Einheit kovalent aneinander gebunden sind, worin die Verbindungs-Einheit in der nativen Glucocerebrosidase nicht zugegen ist.

3. Multimere Proteinstruktur nach Anspruch 2, welche ein Merkmal aufweist, das ausgewählt ist aus der Gruppe, die besteht aus:
(a) einer Glucocerebrosidase-Aktivität, nachdem die multimere Proteinstruktur Humanplasma-Bedingungen für eine Stunde unterworfen wurde, die wenigstens 10 % höher ist als eine Aktivität von nativer Glucocerebrosidase, nachdem die native Glucocerebrosidase den Humanplasma-Bedingungen für eine Stunde unterworfen wurde;
(b) einer Glucocerebrosidase-Aktivität, die sich verringert, nachdem die mulimere Proteinstruktur Humanplasma-Bedingungen für eine Stunde unterworfen wurde, um einen Prozentsatz, der wenigstens 10 % weniger ist als der Prozentsatz, um den sich eine Aktivität der nativen Glucocerebrosidase verringert, nachdem die native Glucocerebrosidase den Humanplasma-Bedingungen für eine Stunde unterworfen wurde;
(c) einer Glucocerebrosidase-Aktivität, die im wesentlich unverändert bleibt, nachdem die multimere Proteinstruktur Humanplasma-Bedingungen für eine Stunde unterworfen wurde;
(d) einer Glucocerebrosidase-Aktivität, nachdem die multimere Proteinstruktur lysosomalen Bedingungen für 4 Tage unterworfen wurde, die wenigstens 10 % höher ist als eine Aktivität von nativer Glucocerebrosidase, nachdem die native Glucocerebrosidase den lysosomalen Bedingungen für 4 Tage unterworfen wurde.
(e) einer Glucocerebrosidase-Aktivität, die sich verringert, wenn die multimere Proteinstruktur lysosomalen Bedingungen für einen Tag unterworfen wurde, um einen Prozentsatz, der wenigstens 10 % weniger ist als der Prozentsatz, um den sich eine Aktivität der nativen Glucocerebrosidase verringert, nachdem die native Glucocerebrosidase den lysosomalen Bedingungen für einen Tag unterworfen wurde;
(f) einer Glucocerebrosidase-Aktivität, die im wesentlichen unverändert bleibt, nachdem die multimere Proteinstruktur lysosomalen Bedingungen für einen Tag unterworfen wurde; und
(g) einer zirkulierenden Halbwertszeit in einem physiologischen System, die um wenigstens 20 % höher ist als eine zirkulierende Halbwertszeit der nativen Glucocerebrosidase.

4. Multimere Proteinstruktur nach irgendeinem der Ansprüche 1 und 3, worin die zirkulierende Halbwertszeit der multimeren Proteinstruktur, die höher ist als eine zirkulierende Halbwertszeit der nativen Glucocerebrosidase, um wenigstens 50 % höher ist als die zirkulierende Halbwertszeit der nativen Glucocerebrosidase.

5. Multimere Proteinstruktur nach irgendeinem der Ansprüche 1, 3 und 4, **gekennzeichnet durch** eine Glucocerebrosidase-Aktivität, nachdem die multimere Proteinstruktur Humanplasma-Bedingungen für eine Stunde unterworfen wurde, die wenigstens das 10-fache einer Aktivität von nativer Glucocerebrosidase ist, nachdem die native Glucocerebrosidase den Humanplasma-Bedingungen für eine Stunde unterworfen wurde.

6. Multimere Proteinstruktur nach irgendeinem der Ansprüche 1 bis 5, **gekennzeichnet durch** eine Glucocerebrosidase-Aktivität in einem Organ nach Verabreichung der multimeren Proteinstruktur an ein Wirbeltier, wobei das Organ ausgewählt ist aus der Gruppe, die besteht aus Leber, Milz, Nieren, Lunge, Knochenmark und Blut.

7. Multimere Proteinstruktur nach irgendeinem der Ansprüche 1 bis 6, die zwei Glucocerebrosidase-Moleküle umfaßt, wobei die Proteinstruktur eine dimere Proteinstruktur ist.

8. Multimere Proteinstruktur nach irgendeinem der Ansprüche 1 bis 7, worin die Glucocerebrosidase eine Aminosäure-Sequenz aufweist, die ausgewählt ist aus der Gruppe, die besteht aus SEQ ID-Nr. 1, SEQ ID-Nr. 2 und SEQ ID-Nr. 3.

9. Multimere Proteinstruktur nach irgendeinem der Ansprüche 1 bis 8, worin die Verbindungs-Einheit Poly(alkylenglykol) umfaßt.

10. Multimere Proteinstruktur nach irgendeinem der Ansprüche 1 bis 8, worin die wenigstens eine Verbindungs-Einheit die allgemeine Formel aufweist:
-X₁-(CR₁R₂-CR₃R₄-Y)ₙ-X₂-
worin jeder der Reste X₁ und X₂ für eine funktionelle Gruppe steht, die eine kovalente Bindung mit wenigstens einem Glucocerebrosidase-Molekül bildet;
Y für O, S oder NR₅ steht;
n für eine ganze Zahl von 1 bis 200 steht; und
jeder der Reste R₁, R₂, R₃, R₄ und R₅ unabhängig voneinander ausgewählt ist aus der Gruppe, die besteht aus Wasserstoff, Alkyl, Cycloalkyl, Alkenyl, Alkinyl, Alkoxy, Hydroxy, Oxo, Thiol und Thioalkoxy.

11. Pharmazeutische Zusammensetzung, die eine multimere Proteinstruktur nach irgendeinem der Ansprüche 1 bis 10 und einen pharmazeutisch annehmbaren Träger umfaßt.

12. Pharmazeutische Zusammensetzung nach Anspruch 11, die darüber hinaus einen Bestandteil umfaßt, der ausgewählt ist aus der Gruppe, die besteht aus Glucose, einem Saccharid, das Glucose-Einheiten umfaßt, Nojirimycin und Derivate davon.

13. Multimere Proteinstruktur nach irgendeinem der Ansprüche 1 bis 10 für die Verwendung bei der Behandlung der Gaucher-Erkrankung.

14. Verfahren zur Herstellung der multimeren Proteinstruktur nach irgendeinem der Ansprüche 1 bis 10, wobei das Verfahren die Schritte umfaßt, daß man Glucocerebrosidase mit einem Vernetzungsmittel umsetzt, welches die Verbindungs-Einheit und wenigstens zwei reaktive Gruppen umfaßt.

15. Verfahren nach Anspruch 14, worin das molare Verhältnis des Vernetzungsmittels zur Glucocerebrosidase im Bereich von 5 : 1 bis 500 : 1 liegt.

## Revendications

1. Structure protéique multimère comprenant au moins deux molécules de glucocérébrosidase liées par liaison covalente les unes aux autres par l'intermédiaire d'un fragment de liaison, la structure protéique multimère présentant une caractéristique choisie dans le groupe constitué :
(a) d'une activité de glucocérébrosidase quand la structure protéique multimère est soumise à des conditions de plasma humain pendant une heure, qui est supérieure d'au moins 10 % à une activité de glucocérébrosidase native quand ladite glucocérébrosidase native est soumise auxdites conditions de plasma humain pendant une heure ;
(b) d'une activité de glucocérébrosidase qui diminue quand la structure protéique multimère est soumise à des conditions de plasma humain pendant une heure d'un pourcentage qui est inférieur d'au moins 10 % au pourcentage duquel une activité de ladite glucocérébrosidase native diminue quand ladite glucocérébrosidase native est soumise auxdites conditions de plasma humain pendant une heure ;
(c) d'une activité de glucocérébrosidase qui reste sensiblement inchangée quand la structure protéique multimère est soumise à des conditions de plasma humain pendant une heure ;
(d) d'une activité de glucocérébrosidase, quand la structure protéique multimère est soumise à des conditions lysosomiales pendant 4 jours, qui est supérieure d'au moins 10 % à une activité de glucocérébrosidase native quand ladite glucocérébrosidase native est soumise auxdites conditions lysosomiales pendant 4 jours ;
(e) d'une activité de glucocérébrosidase qui diminue quand la structure protéique multimère est soumise à des conditions lysosomiales pendant un jour d'un pourcentage qui est inférieur d'au moins 10 % au pourcentage duquel une activité de ladite glucocérébrosidase native diminue quand ladite glucocérébrosidase native est soumise auxdites conditions lysosomiales pendant un jour ;
(f) d'une activité de glucocérébrosidase qui reste sensiblement inchangée quand la structure protéique multimère est soumise à des conditions lysosomiales pendant un jour ; et
(g) d'une demi-vie en circulation dans un système physiologique qui est supérieure d'au moins 20 % à une demi-vie en circulation de ladite glucocérébrosidase native.

2. Structure protéique multimère comprenant au moins deux molécules de glucocérébrosidase liées par liaison covalente les unes aux autres par l'intermédiaire d'un fragment de liaison, dans laquelle ledit fragment de liaison n'est pas présent dans la glucocérébrosidase native.

3. Structure protéique multimère selon la revendication 2, présentant une caractéristique choisie dans le groupe constitué :
(a) d'une activité de glucocérébrosidase quand la structure protéique multimère est soumise à des conditions de plasma humain pendant une heure, qui est supérieure d'au moins 10 % à une activité de glucocérébrosidase native quand ladite glucocérébrosidase native est soumise auxdites conditions de plasma humain pendant une heure ;
(b) d'une activité de glucocérébrosidase qui diminue quand la structure protéique multimère est soumise à des conditions de plasma humain pendant une heure d'un pourcentage qui est inférieur d'au moins 10 % au pourcentage duquel une activité de ladite glucocérébrosidase native diminue quand ladite glucocérébrosidase native est soumise auxdites conditions de plasma humain pendant une heure ;
(c) d'une activité de glucocérébrosidase qui reste sensiblement inchangée quand la structure protéique multimère est soumise à des conditions de plasma humain pendant une heure ;
(d) d'une activité de glucocérébrosidase, quand la structure protéique multimère est soumise à des conditions lysosomiales pendant 4 jours, qui est supérieure d'au moins 10 % à une activité de glucocérébrosidase native quand ladite glucocérébrosidase native est soumise auxdites conditions lysosomiales pendant 4 jours ;
(e) d'une activité de glucocérébrosidase qui diminue quand la structure protéique multimère est soumise à des conditions lysosomiales pendant un jour d'un pourcentage qui est inférieur d'au moins 10 % au pourcentage duquel une activité de ladite glucocérébrosidase native diminue quand ladite glucocérébrosidase native est soumise auxdites conditions lysosomiales pendant un jour ;
(f) d'une activité de glucocérébrosidase qui reste sensiblement inchangée quand la structure protéique multimère est soumise à des conditions lysosomiales pendant un jour ; et
(e) d'une demi-vie en circulation dans un système physiologique qui est supérieure d'au moins 20 % à une demi-vie en circulation de ladite glucocérébrosidase native.

4. Structure protéique multimère selon l'une quelconque des revendications 1 et 3, dans laquelle ladite demi-vie en circulation de la structure protéique multimère qui est supérieure à une demi-vie en circulation de ladite glucocérébrosidase native, est supérieure d'au moins 50 % à ladite demi-vie en circulation de ladite glucocérébrosidase native.

5. Structure protéique multimère selon l'une quelconque des revendications 1, 3 et 4, qui est **caractérisée par** une activité de glucocérébrosidase quand la structure protéique multimère est soumise à des conditions de plasma humain pendant une heure, qui représente au moins 10 fois une activité de glucocérébrosidase native quand ladite glucocérébrosidase native est soumise auxdites conditions de plasma humain pendant une heure.

6. Structure protéique multimère selon l'une quelconque des revendications 1 à 5, **caractérisée par** une activité de glucocérébrosidase dans un organe lors de l'administration de la ladite structure protéique multimère à un vertébré, ledit organe étant choisi dans le groupe constitué d'un foie, d'une rate, d'un rein, d'un poumon, d'une moelle osseuse et du sang.

7. Structure protéique multimère selon l'une quelconque des revendications 1 à 6, comprenant deux molécules de glucocérébrosidase, la structure protéique étant une structure protéique dimère.

8. Structure protéique multimère selon l'une quelconque des revendications 1 à 7, dans laquelle ladite glucocérébrosidase possède une séquence d'acides aminés choisie dans le groupe constitué de SEQ ID NO : 1, SEQ ID NO : 2 et SEQ ID NO : 3.

9. Structure protéique multimère selon l'une quelconque des revendications 1 à 8, dans laquelle ledit fragment de liaison comprend un poly(alkylène glycol).

10. Structure protéique multimère selon l'une quelconque des revendications 1 à 8, dans laquelle ledit au moins un fragment de liaison possède une formule général :
-X₁-(CR₁R₂-CR₃R₄-Y) n-X₂-
dans laquelle X₁ et X₂ sont chacun un groupe fonctionnel qui forme une liaison covalente avec au moins une molécule de glucocérébrosidase ;
Y est 0, S ou NR₅ ;
n est un nombre entier allant de 1 à 200 ; et
R₁, R₂, R₃, R₄ et R₅ sont chacun choisis indépendamment dans le groupe constitué d'un hydrogène, d'un alkyle, d'un cycloalkyle, d'un alcényle, d'un alcynyle, d'un alcoxy, d'un hydroxy, d'un oxo, d'un thiol et d'un thioalcoxy.

11. Composition pharmaceutique comprenant la structure protéique multimère selon l'une quelconque des revendications 1 à 10 et un support pharmaceutiquement acceptable.

12. Composition pharmaceutique selon la revendication 11, comprenant en outre un ingrédient choisi dans le groupe constitué du glucose, d'un saccharide comprenant un fragment de glucose, de la nojirimycine et des dérivés de ceux-ci.

13. Structure protéique multimère selon l'une quelconque des revendications 1 à 10, pour une utilisation dans le traitement de la maladie de Gaucher.

14. Procédé de préparation de la structure protéique multimère selon l'une quelconque des revendications 1 à 10, le procédé comprenant la réaction d'une glucocérébrosidase avec un agent de réticulation qui comprend ledit fragment de liaison et au moins deux groupes réactifs.

15. Procédé selon la revendication 14, dans lequel un rapport molaire entre ledit agent de réticulation et ladite glucocérébrosidase est situé dans une plage allant de 5 : 1 à 500 : 1.


### SEQUENCE LISTING

<110> Protalix Ltd. Ruderfer , Ilya Kizhner, Tali Shulman, Avidor Shaaltiel, Yoseph
<120> GLUCOCEREBROSIDASE MULTIMERS AND USES THEREOF
<130> 50864
<150> US 61/309,487
   <151> 2010-03-02
<160> 3
<170> PatentIn version 3.5
<210> 1
   <211> 497
   <212> PRT
   <213> Artificial sequence
<220>
   <223> Imiglucerase protein sequence
<400> 1
<210> 2
   <211> 497
   <212> PRT
   <213> Homo sapiens
<400> 2
   Ala Arg Pro Cys Ile Pro Lys Ser Phe Gly Tyr Ser Ser Val Val Cys
   1               5                   10                  15
   Val Cys Asn Ala Thr Tyr Cys Asp Ser Phe Asp Pro Pro Thr Phe Pro
               20                  25                  30
   Ala Leu Gly Thr Phe Ser Arg Tyr Glu Ser Thr Arg Ser Gly Arg Arg
           35                  40                  45
   Met Glu Leu Ser Met Gly Pro Ile Gln Ala Asn His Thr Gly Thr Gly
       50                  55                  60
   Leu Leu Leu Thr Leu Gln Pro Glu Gln Lys Phe Gln Lys Val Lys Gly
   65                  70                  75                  80
   Phe Gly Gly Ala Met Thr Asp Ala Ala Ala Leu Asn Ile Leu Ala Leu
                   85                  90                  95
   Ser Pro Pro Ala Gln Asn Leu Leu Leu Lys Ser Tyr Phe Ser Glu Glu
               100                 105                 110
   Gly Ile Gly Tyr Asn Ile Ile Arg Val Pro Met Ala Ser Cys Asp Phe
           115                 120                 125
   Ser Ile Arg Thr Tyr Thr Tyr Ala Asp Thr Pro Asp Asp Phe Gln Leu
       130                 135                 140
   His Asn Phe Ser Leu Pro Glu Glu Asp Thr Lys Leu Lys Ile Pro Leu
   145                 150                 155                 160
   Ile His Arg Ala Leu Gln Leu Ala Gln Arg Pro Val Ser Leu Leu Ala
                   165                 170                 175
   Ser Pro Trp Thr Ser Pro Thr Trp Leu Lys Thr Asn Gly Ala Val Asn
               180                 185                 190
   Gly Lys Gly Ser Leu Lys Gly Gln Pro Gly Asp Ile Tyr His Gln Thr
           195                 200                 205
   Trp Ala Arg Tyr Phe Val Lys Phe Leu Asp Ala Tyr Ala Glu His Lys
       210                 215                 220
   Leu Gln Phe Trp Ala Val Thr Ala Glu Asn Glu Pro Ser Ala Gly Leu
   225                 230                 235                 240
   Leu Ser Gly Tyr Pro Phe Gln Cys Leu Gly Phe Thr Pro Glu His Gln
   245 250 255
   Arg Asp Phe Ile Ala Arg Asp Leu Gly Pro Thr Leu Ala Asn Ser Thr
   260 265 270
   His His Asn Val Arg Leu Leu Met Leu Asp Asp Gln Arg Leu Leu Leu
   275 280 285
   Pro His Trp Ala Lys Val Val Leu Thr Asp Pro Glu Ala Ala Lys Tyr
   290 295 300
   Val His Gly Ile Ala Val His Trp Tyr Leu Asp Phe Leu Ala Pro Ala
   305 310 315 320
   Lys Ala Thr Leu Gly Glu Thr His Arg Leu Phe Pro Asn Thr Met Leu
   325 330 335
   Phe Ala Ser Glu Ala Cys Val Gly Ser Lys Phe Trp Glu Gln Ser Val
   340 345 350
   Arg Leu Gly Ser Trp Asp Arg Gly Met Gln Tyr Ser His Ser Ile Ile
   355 360 365
   Thr Asn Leu Leu Tyr His Val Val Gly Trp Thr Asp Trp Asn Leu Ala
   370 375 380
   Leu Asn Pro Glu Gly Gly Pro Asn Trp Val Arg Asn Phe Val Asp Ser
   385 390 395 400
   Pro Ile Ile Val Asp Ile Thr Lys Asp Thr Phe Tyr Lys Gln Pro Met
   405 410 415
   Phe Tyr His Leu Gly His Phe Ser Lys Phe Ile Pro Glu Gly Ser Gln
   420 425 430
   Arg Val Gly Leu Val Ala Ser Gln Lys Asn Asp Leu Asp Ala Val Ala
   435 440 445
   Leu Met His Pro Asp Gly Ser Ala Val Val Val Val Leu Asn Arg Ser
   450 455 460
   Ser Lys Asp Val Pro Leu Thr Ile Lys Asp Pro Ala Val Gly Phe Leu
   465 470 475 480
   Glu Thr Ile Ser Pro Gly Tyr Ser Ile His Thr Tyr Leu Trp Arg Arg
   485 490 495
   Gln
<210> 3
   <211> 506
   <212> PRT
   <213> Artificial sequence
<220>
   <223> Taliglucerase protein sequence
<400> 3
   Glu Phe Ala Arg Pro Cys Ile Pro Lys Ser Phe Gly Tyr Ser Ser Val
   1               5                   10                  15
   Val Cys Val Cys Asn Ala Thr Tyr Cys Asp Ser Phe Asp Pro Pro Thr
               20                  25                  30
   Phe Pro Ala Leu Gly Thr Phe Ser Arg Tyr Glu Ser Thr Arg Ser Gly
           35                  40                  45
   Arg Arg Met Glu Leu Ser Met Gly Pro Ile Gln Ala Asn His Thr Gly
       50                  55                  60
   Thr Gly Leu Leu Leu Thr Leu Gln Pro Glu Gln Lys Phe Gln Lys Val
   65                  70                  75                  80
   Lys Gly Phe Gly Gly Ala Met Thr Asp Ala Ala Ala Leu Asn Ile Leu
                   85                  90                  95
   Ala Leu Ser Pro Pro Ala Gln Asn Leu Leu Leu Lys Ser Tyr Phe Ser
               100                 105                 110
   Glu Glu Gly Ile Gly Tyr Asn Ile Ile Arg Val Pro Met Ala Ser Cys
           115                 120                 125
   Asp Phe Ser Ile Arg Thr Tyr Thr Tyr Ala Asp Thr Pro Asp Asp Phe
       130                 135                 140
   Gln Leu His Asn Phe Ser Leu Pro Glu Glu Asp Thr Lys Leu Lys Ile
   145                 150                 155                 160
   Pro Leu Ile His Arg Ala Leu Gln Leu Ala Gln Arg Pro Val Ser Leu
                   165                 170                 175
   Leu Ala Ser Pro Trp Thr Ser Pro Thr Trp Leu Lys Thr Asn Gly Ala
               180                 185                 190
   Val Asn Gly Lys Gly Ser Leu Lys Gly Gln Pro Gly Asp Ile Tyr His
           195                 200                 205
   Gln Thr Trp Ala Arg Tyr Phe Val Lys Phe Leu Asp Ala Tyr Ala Glu
       210                 215                 220
   His Lys Leu Gln Phe Trp Ala Val Thr Ala Glu Asn Glu Pro Ser Ala
   225                 230                 235                 240
   Gly Leu Leu Ser Gly Tyr Pro Phe Gln Cys Leu Gly Phe Thr Pro Glu
                   245                 250                 255
   His Gln Arg Asp Phe Ile Ala Arg Asp Leu Gly Pro Thr Leu Ala Asn
               260                 265                 270
   Ser Thr His His Asn Val Arg Leu Leu Met Leu Asp Asp Gln Arg Leu
           275                 280                 285
   Leu Leu Pro His Trp Ala Lys Val Val Leu Thr Asp Pro Glu Ala Ala
       290                 295                 300
   Lys Tyr Val His Gly Ile Ala Val His Trp Tyr Leu Asp Phe Leu Ala
   305                 310                 315                 320
   Pro Ala Lys Ala Thr Leu Gly Glu Thr His Arg Leu Phe Pro Asn Thr
                   325                 330                 335
   Met Leu Phe Ala Ser Glu Ala Cys Val Gly Ser Lys Phe Trp Glu Gln
               340                 345                 350
   Ser Val Arg Leu Gly Ser Trp Asp Arg Gly Met Gln Tyr Ser His Ser
           355                 360                 365
   Ile Ile Thr Asn Leu Leu Tyr His Val Val Gly Trp Thr Asp Trp Asn
       370                 375                 380
   Leu Ala Leu Asn Pro Glu Gly Gly Pro Asn Trp Val Arg Asn Phe Val
   385                 390                 395                 400
   Asp Ser Pro Ile Ile Val Asp Ile Thr Lys Asp Thr Phe Tyr Lys Gln
                   405                 410                 415
   Pro Met Phe Tyr His Leu Gly His Phe Ser Lys Phe Ile Pro Glu Gly
               420                 425                 430
   Ser Gln Arg Val Gly Leu Val Ala Ser Gln Lys Asn Asp Leu Asp Ala
           435                 440                 445
   Val Ala Leu Met His Pro Asp Gly Ser Ala Val Val Val Val Leu Asn
       450                 455                 460
   Arg Ser Ser Lys Asp Val Pro Leu Thr Ile Lys Asp Pro Ala Val Gly
   465                 470                 475                 480
   Phe Leu Glu Thr Ile Ser Pro Gly Tyr Ser Ile His Thr Tyr Leu Trp
                   485                 490                 495
   His Arg Gln Asp Leu Leu Val Asp Thr Met
               500                 505